# EUROPEAN PATENT APPLICATION

(11) **EP 4 831 269 A1**
(43) Date of publication of application: **28.10.2026**
(21) Application number: 24906809.9
(22) Date of filing: 06.08.2024
(51) Int. Cl.: C12M 1/00, C12M 3/00

(54) **CELL CULTURE APPARATUS**

(30) Priority: 20.12.2023 JP 2023214803
(71) Applicant: Nikkiso Co., Ltd., Tokyo 150-6022 (JP)
(72) Inventor: NASU, Keita, Higashimurayama-shi, Tokyo 189-8520 (JP); MORI, Takahiro, Higashimurayama-shi, Tokyo 189-8520 (JP); MORINAGA, Takahiro, Higashimurayama-shi, Tokyo 189-8520 (JP); IWASAKI, Tatsuya, Higashimurayama-shi, Tokyo 189-8520 (JP)
(74) Representative: Mathys & Squire
(86) International application number: PCT/JP2024/027994
(87) International publication number: WO 2025/134419

(57) **Abstract**

Provided is a cell culture apparatus capable of operating operation portions having different functions in a more suitable environment. The cell culture apparatus includes a first space (such as an internal space of a culture medium exchange portion 22) having air cleanliness that satisfies a first criterion (such as Class 5 according to ISO) as a standard criterion thereof; a second space (such as an internal space of a portion other than the culture medium exchange portion 22) having air cleanliness that satisfies a second criterion (such as Classes 6 to 7 according to ISO) as a standard criterion thereof, the second criterion being lower than the first criterion; a culture medium exchange robot (42) that is provided in the first space, the culture medium exchange robot being configured to execute an operation related to culture medium exchange; a transfer robot (40) that is provided in the second space, the transfer robot being configured to execute a moving operation of a well plate (52) that stores a culture medium; and an operation portion controller that is configured to set, as the first space, a movable range in a case where the culture medium exchange robot (42) executes the operation related to the culture medium exchange, and set, as the second space and the first space, a movable range in a case where the transfer robot (40) moves the well plate (52).

## Description

### Technical Field

The present invention relates to, for example, a cell culture apparatus including a plurality of operation robots.

### Background Art

Patent Literature 1 (claim 1 and the like) listed below discloses an invention related to an automatic culture operation device in which a first robot and a second robot are disposed in a working chamber so that movable ranges of the first robot and the second robot partially overlap each other. In Patent Literature 1, a liquid supply means and a temporary placement portion are provided in overlapping movable ranges of the first robot and the second robot. Then, the liquid supply means supplies liquids such as a culture medium and a chemical solution to the containers held by the robot, and the first robot and the second robot transfer the containers in the temporary placement portion.

### Citation List

### Patent Literature

Patent Literature 1: JP 6358429 B2

### Summary of Invention

### Technical Problem

Meanwhile, Patent Literature 1 describes that purified air is circulated to maintain a positive pressure in the working chamber. However, Patent Literature 1 does not describe that an installation environment and a working environment for operation portions having different functions, such as the first robot and the second robot, are further optimized.

An object of the present invention is to provide a cell culture apparatus capable of operating operation portions having different functions in a more optimized environment.

### Solution to Problem

A cell culture apparatus according to an embodiment of the present invention includes:
a first space having air cleanliness that satisfies a first criterion as a standard criterion thereof;
a second space having air cleanliness that satisfies a second criterion as a standard criterion thereof, the second criterion being lower than the first criterion;
a first operation portion that is provided in the first space, the first operation portion being configured to execute an operation related to culture medium exchange;
a second operation portion that is provided in the second space, the second operation portion being configured to execute a moving operation of a cell culture container that stores a culture medium; and
an operation portion controller that is configured to
set, as the first space, a movable range in a case where the first operation portion executes the operation related to the culture medium exchange, and
set, as the second space and the first space, a movable range in a case where the second operation portion moves the cell culture container.

### Effects of Invention

According to the present invention, it is possible to provide the cell culture apparatus capable of operating the operation portions having different functions in a more optimized environment.

### Brief Description of Drawings

[FIG. 1] FIG. 1 is a perspective view illustrating a cell culture apparatus according to an embodiment.
[FIG. 2] FIG. 2 is a perspective view illustrating the cell culture apparatus in a state where a wall portion is removed.
[FIG. 3] FIG. 3 is an explanatory view illustrating a layout of portions.
[FIG. 4] (a) of FIG. 4 is an explanatory view illustrating another explanatory mode of the layout, and (b) of FIG. 4 is an explanatory view illustrating still another explanatory mode of the layout.
[FIG. 5] FIG. 5 is an explanatory view illustrating a basic configuration of the portions.
[FIG. 6] FIG. 6 is a perspective view illustrating well plates and a stocker.
[FIG. 7] (a) of FIG. 7 is a perspective view illustrating an air gripper, and (b) of FIG. 7 is an explanatory view illustrating an operation of gripping a stocker by a grip portion.
[FIG. 8] FIG. 8 is an explanatory view illustrating an orientation of a stocker.
[FIG. 9] (a) of FIG. 9 is an explanatory view illustrating an image inspection portion in plan view, and (b) of FIG. 9 is an explanatory view illustrating the image inspection portion in side view.
[FIG. 10] FIG. 10 is an explanatory view illustrating a culture medium exchange portion.
[FIG. 11] FIG. 11 is an explanatory view illustrating a relationship between a culture medium exchange portion and a transfer robot.
[FIG. 12] FIG. 12 is an explanatory view illustrating a modification of the layout of the portions.
[FIG. 13] FIG. 13 is an explanatory view illustrating a modification of arrangement of a culture medium exchange robot.
[FIG. 14] FIG. 14 is a perspective view illustrating a micropipette tool to which a multi-channel micropipette is attached.
[FIG. 15] FIG. 15 is a perspective view illustrating a pipettor tool to which an electric pipettor is attached.
[FIG. 16] (a) of FIG. 16 is an explanatory view illustrating a state before a distal end bit is attached to a multi-aspirator, and (b) of FIG. 16 is an explanatory view illustrating a state after the distal end bit is attached to the multi-aspirator.
[FIG. 17] (a) of FIG. 17 is an explanatory view illustrating a state in which a fall prevention plate advances, and (b) of FIG. 17 is an explanatory view illustrating a state in which the multi-aspirator to which the distal end bit is attached rises.
[FIG. 18] FIG. 18 is an explanatory view illustrating a state in which the fall prevention plate advances.
[FIG. 19] (a) of FIG. 19 is an explanatory view illustrating, from a side, a state in which the presence or absence of falling of a distal end bit 142 is detected, and (b) of FIG. 19 is an explanatory view illustrating, from a traveling direction, a state in which the presence or absence of falling of the distal end bit 142 is detected.
[FIG. 20] (a) of FIG. 20 is an explanatory view illustrating, from obliquely above, a state in which a force for attaching a pipette tip to the pipettor tool is assisted, and (b) of FIG. 20 is an explanatory view illustrating, from a side, a state in which the force for attaching the pipette tip to the pipettor tool is assisted.
[FIG. 21] (a) of FIG. 21 is an explanatory view illustrating, from obliquely above, a state in which an automatic door of an incubator is closed, and (b) of FIG. 21 is an explanatory view illustrating, from obliquely above, a state in which the automatic door of the incubator is opened.
[FIG. 22] FIG. 22 is an explanatory view illustrating, from obliquely above, a state in which an inner door of the incubator is opened.
[FIG. 23] (a) of FIG. 23 is an explanatory view illustrating, from obliquely above, a part of an upper side of the automatic door in the incubator, and (b) of FIG. 23 is an explanatory view illustrating, from obliquely below, a part of a lower side of the automatic door in the incubator.
[FIG. 24] (a) of FIG. 24 is an explanatory view illustrating, from obliquely above, a state in which two automatic opening and closing shutters of the culture medium exchange portion facing a second space are closed together, and (b) of FIG. 24 is an explanatory view illustrating, from obliquely above, a state in which one automatic opening and closing shutter of the culture medium exchange portion is fully opened.
[FIG. 25] (a) of FIG. 25 is an explanatory view illustrating, from obliquely above, a state in which the other automatic opening and closing shutter of the culture medium exchange portion is fully opened, and (b) of FIG. 25 is an explanatory view illustrating, from obliquely above, a state in which the one automatic opening and closing shutter of the culture medium exchange portion is half-opened.
[FIG. 26] FIG. 26 is an explanatory view illustrating a shaking operation for gripping one reservoir.
[FIG. 27] (a) of FIG. 27 is an explanatory view illustrating, from a side, a state in which a disposal shutter is closed, and (b) of FIG. 27 is an explanatory view illustrating, from the side, a state in which the disposal shutter is opened.
[FIG. 28] FIG. 28 is an explanatory view illustrating downflow of air in a first space.
[FIG. 29] FIG. 29 is an explanatory view illustrating a state in which one external disposal port is opened in a wall portion outside the culture medium exchange portion.
[FIG. 30] FIG. 30 is an explanatory view illustrating a waste liquid tube of the culture medium exchange robot.

### Description of Embodiments

### <Basic configuration of cell culture apparatus 10>

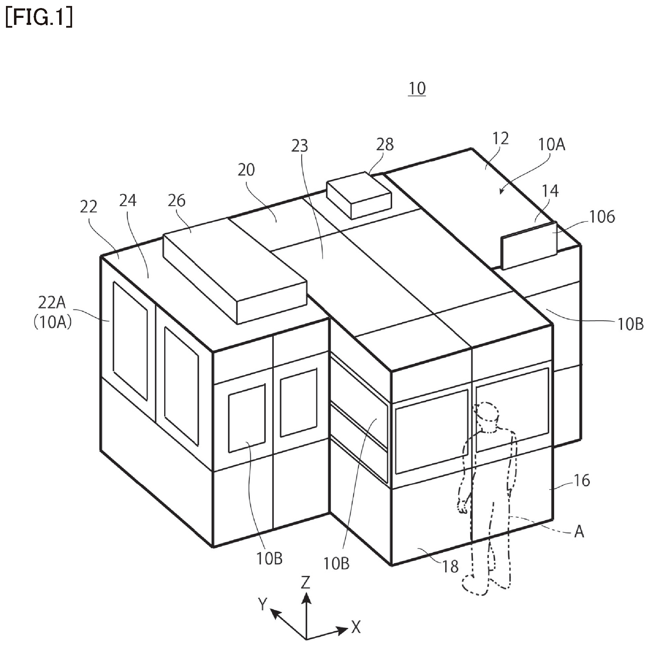

Hereinafter, a cell culture apparatus 10 according to an embodiment will be described with reference to the drawings. FIG. 1 illustrates the cell culture apparatus 10 according to the embodiment. The cell culture apparatus 10 includes an incubator portion 12, an image inspection portion 14, a stocker supply portion 16, a product removal portion 18, a transfer robot portion 20, and a culture medium exchange portion 22. Hereinafter, these may be collectively referred to as "the portions".

Further, in the present embodiment, the stocker supply portion 16 and the product removal portion 18 are integrated. Thus, for example, both can be collectively referred to as a "stocker supply and removal portion" or a "stocker supply and discharge portion".

A portion of about 2/3 from the top of each portion is surrounded by a wall portion 10A. In the present embodiment, the wall portion 10A is formed in a T shape in a plan view of the cell culture apparatus 10 from the ceiling side, and airtightly surrounds the inner space. The wall portion 10A is provided with a plurality of transparent window portions 10B through which an operator A (indicated by a two-dot chain line) or the like can visually observe the portions. Note that the arrangement of the wall portion 10A is not limited to the T-shape, and may be variously changed to a rectangular shape, an L-shape, or the like depending on circumstances such as the shape of the installation space, for example.

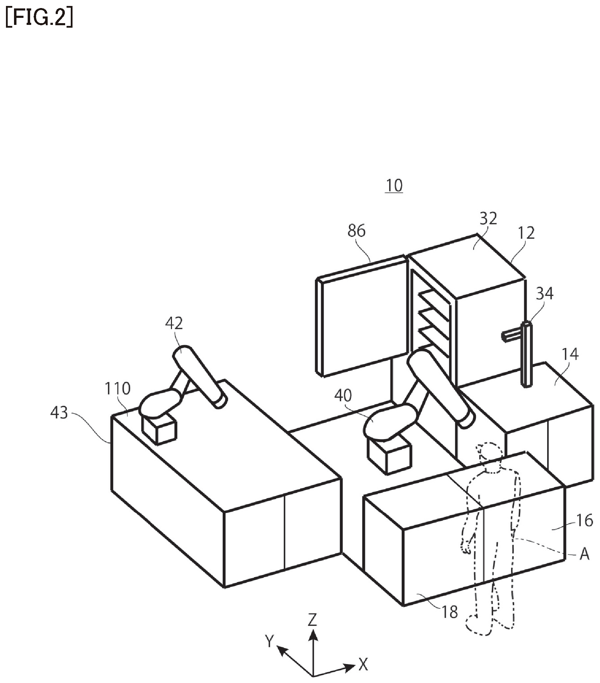

FIG. 2 illustrates a state in which the wall portion 10A is removed from the cell culture apparatus 10 of FIG. 1. As illustrated in FIG. 2, the cell culture apparatus 10 is provided with an incubator 32, an image inspection apparatus 34, a transfer robot 40, and a culture medium exchange robot 42. The culture medium exchange robot 42 is provided in a medium exchange device 43. Note that details of these devices and the portions will be described later.

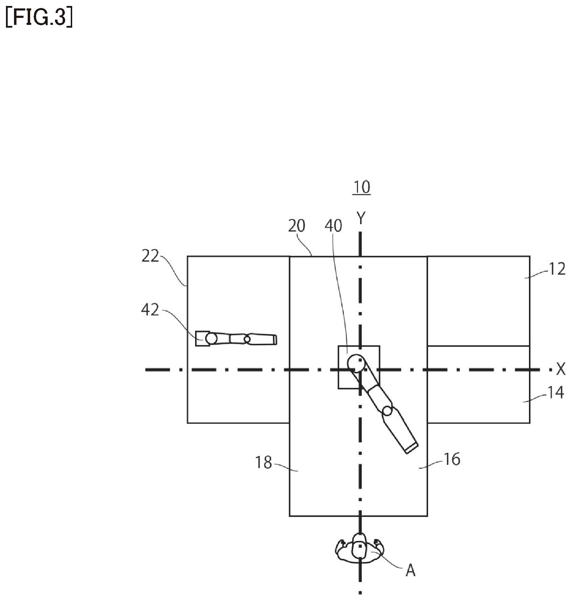

FIG. 3 illustrates a layout of the portions. More specifically, FIG. 3 illustrates a state in which the wall portion 10A of the cell culture apparatus 10 is removed and viewed in a plan view from above. The transfer robot portion 20 among the portions is disposed in a central portion, and the other portions, namely, the incubator portion 12, the image inspection portion 14, the stocker supply portion 16, the product removal portion 18, and the culture medium exchange portion 22 are sequentially disposed so as to surround the transfer robot portion 20 in a clockwise direction in FIG. 3. The layout of the portions may be described in a plurality of other modes, but other explanatory modes will be described later.

### <First space and second space>

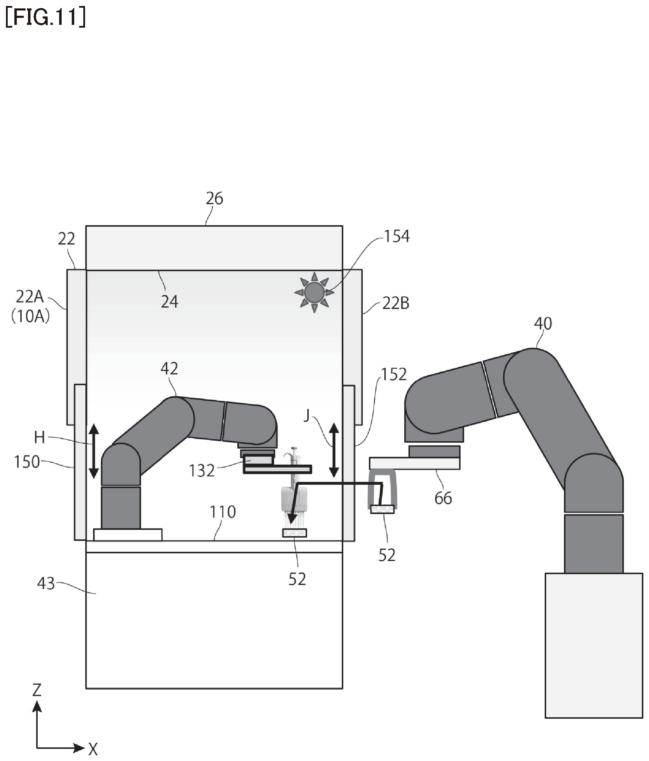

The inside of the cell culture apparatus 10 is divided into an internal space of the culture medium exchange portion 22 and internal spaces of the portions (incubator portion 12, image inspection portion 14, stocker supply portion 16, product removal portion 18, and transfer robot portion 20) other than the culture medium exchange portion 22. The internal spaces of the portions other than the culture medium exchange portion 22 are connected in spatial communication with one another. The internal space of the culture medium exchange portion 22 is defined as a first space, and the internal spaces of the portions other than the culture medium exchange portion 22 are defined as a second space. Inside the wall portion 10A (FIG. 1), as illustrated in FIG. 11, a wall portion 22B as a part of the culture medium exchange portion 22 divides the inside into a first space (an internal space of the culture medium exchange portion 22) and a second space (an internal space other than the culture medium exchange portion 22), as will be described in detail later.

When the cell culture apparatus 10 is used, air cleanliness of the first and second space may be made different from each other. The first space satisfies a first criterion as a standard criterion of air cleanliness, and the second space satisfies a second criterion lower than the first criterion.

In the present embodiment, the first criterion related to the first space is, for example, Class 5 according to the International Organization for Standardization (ISO) (equivalent to Class 100 according to the US Federal Standard) or less. On the other hand, the second criterion related to the second space is, for example, Classes 6 to 7 according to ISO (equivalent to Classes 1000 to 10,000 according to the US Federal Standard). Note that the relationship between the air cleanliness of the first space and the air cleanliness of the second space is not limited to the above example as long as the air cleanliness of the culture medium exchange portion may be maintained to an extent necessary for culture medium exchange. For example, the air cleanliness of the second space may be lowered by three ranks or more as compared with the first space.

As illustrated in FIG. 1, a first space filter unit 26 is provided outside a ceiling portion 24 in the culture medium exchange portion 22. Purified air (air that has been cleaned) is supplied into the first space by the first space filter unit 26, and air cleanliness of the first space is maintained. A second space filter unit 28 is provided outside a ceiling portion 23 in the transfer robot portion 20. Purified air is supplied into the second space by the second space filter unit 28, and air cleanliness of the second space is maintained.

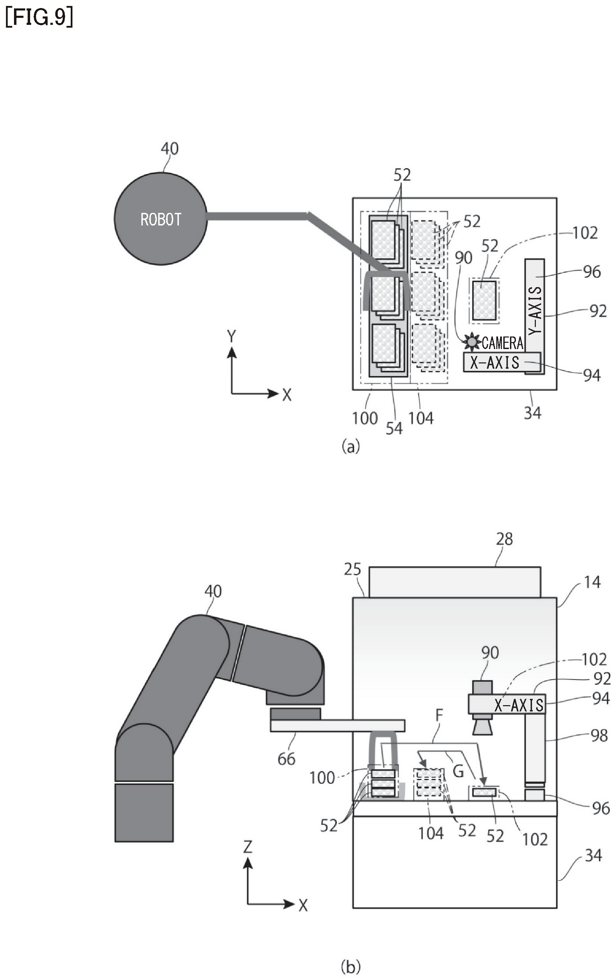

Note that the arrangement of the second space filter unit 28 is not limited to the ceiling portion 23 of the transfer robot portion 20 and may be a ceiling portion of another portion. In addition, the second space filter unit 28 may be disposed at various places. For example, (b) of FIG. 9 schematically illustrates the image inspection portion 14 in a state where the second space filter unit 28 is disposed on the ceiling portion 25 of the image inspection portion 14.

A stocker 54 to be described later is supplied from the stocker supply portion 16 to the first space in which a portion other than the culture medium exchange portion 22 is disposed. Further, a well plate 52 (described later) containing cultured cells is taken out from the product removal portion 18 in a state of being placed on the stocker 54. Note that supply and removal of the stocker 54 can be performed by a transfer robot (not illustrated) installed outside the cell culture apparatus 10. At this time, it is possible to perform the operation in a state where the window portion 10B (which may be a manual door or an automatic door) facing the stocker supply portion 16 and the product removal portion 18 is opened.

In the first space related to the culture medium exchange portion 22, various operations related to culture medium exchange as described later are performed under an air cleanliness environment higher than that of the second space related to the other portions. Note that depending on the process, an automatic opening and closing shutter 152 (FIG. 11, described later) located at the boundary between the culture medium exchange portion 22 and the second space may be opened, and a distal end portion of the transfer robot 40 installed in the first space may enter the second space to perform a predetermined operation.

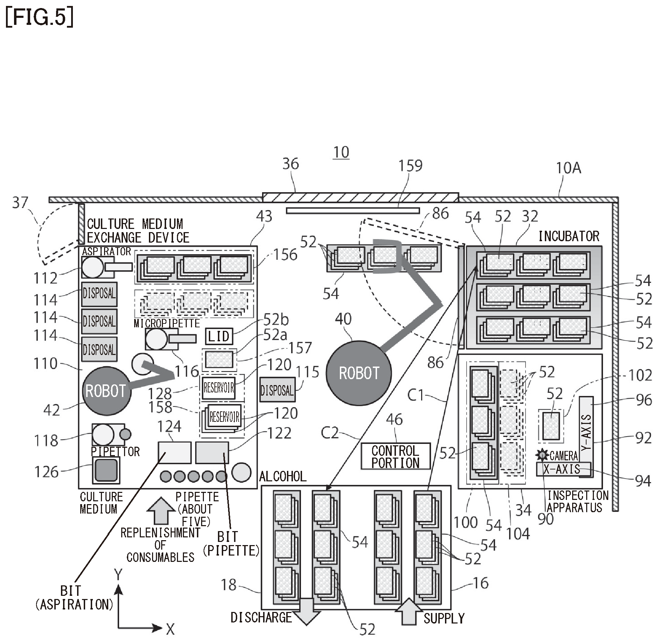

Further, the transfer robot portion 20 in which the transfer robot 40 is installed includes an opening and closing shutter portion 36 (FIG. 5) that divides the second space and a space (external space) outside the cell culture apparatus 10. Then, depending on the situation, the opening and closing shutter portion 36 of the transfer robot portion 20 may be opened to allow the operator A or the like to enter or exit. The operator A is allowed to enter and exit only during maintenance of the incubator 32 and the transfer robot 40.

### <First operation portion and second operation portion>

The cell culture apparatus 10 includes a first operation portion and a second operation portion. The first operation portion is installed in the first space and can execute an operation related to culture medium exchange. The first operation portion corresponds to the culture medium exchange robot 42. The second operation portion is installed in the second space and can execute a moving operation of a cell culture container in which the culture medium is stored. The second operation portion corresponds to the transfer robot 40.

The culture medium exchange robot 42 and the transfer robot 40 are vertical articulated robots having six axes (3 orthogonal axes and rotations about respective axes) of freedom. Note that the culture medium exchange robot 42 and/or the transfer robot 40 may be an articulated robot having less than six axes or more than six axes. Further, the drive systems of the culture medium exchange robot 42 and the transfer robot 40 are both an AC (direct current) servo system. The culture medium exchange robot 42 and the transfer robot 40 are installed at fixed positions of the culture medium exchange portion 22 and the transfer robot portion 20, respectively to move (including rotational movement) distal end portions thereof within respective movable ranges.

The culture medium exchange robot 42 (first operation portion) and the transfer robot 40 (second operation portion) are controlled by the control portion 46 (FIG. 5). The control portion 46 comprehensively represents a control portion of a portion accompanied by computer control among the portions (incubator portion 12, image inspection portion 14, stocker supply portion 16, product removal portion 18, transfer robot portion 20, and culture medium exchange portion 22). The control portion 46 may include a control portion that integrally controls a plurality of portions of the portions.

The control portion 46 has a function of performing control on the culture medium exchange robot 42 (first operation portion) and the transfer robot 40 (second operation portion) so that a movable range in a case where the culture medium exchange robot 42 performs an operation related to the culture medium exchange is set to the first space, and a movable range in a case where the transfer robot 40 moves the cell culture container (well plate 52, described below) is set to the second space and the first space. The control portion of the culture medium exchange robot 42 and the control portion of the transfer robot 40 may be collectively referred to as, for example, an operation portion controller (operation portion control portion) and the like.

Specifically, in the cell culture apparatus 10, the movable range of the culture medium exchange robot 42 at the time of culture medium exchange is limited to the inside of the culture medium exchange portion 22. On the other hand, the movable range of the transfer robot 40 is not limited to the inside of the transfer robot portion 20 and includes the culture medium exchange portion 22 (here, a part of the culture medium exchange portion 22), the incubator portion 12, the image inspection portion 14, the stocker supply portion 16, and the product removal portion 18. Then, the transfer robot 40 can cause the distal end portion to enter any of the portions.

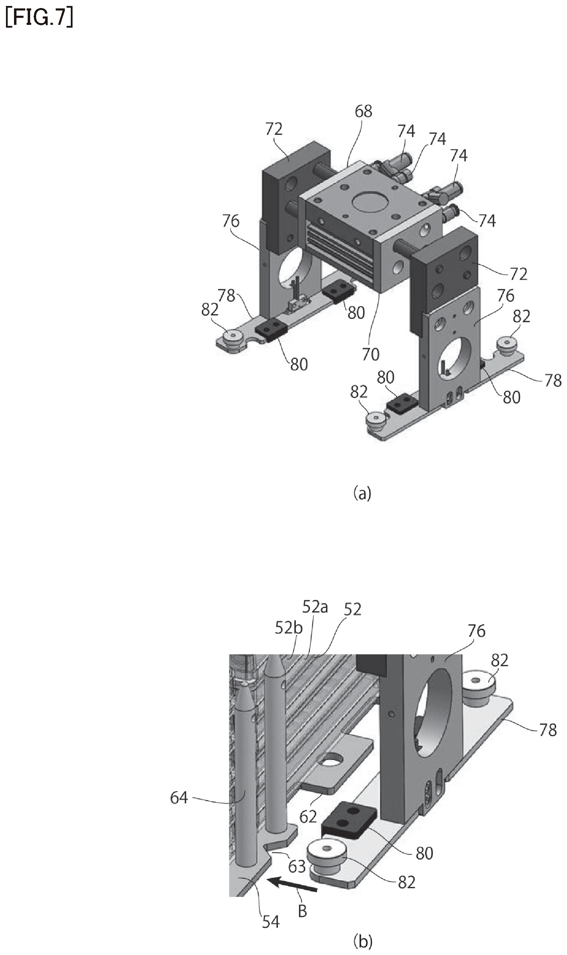

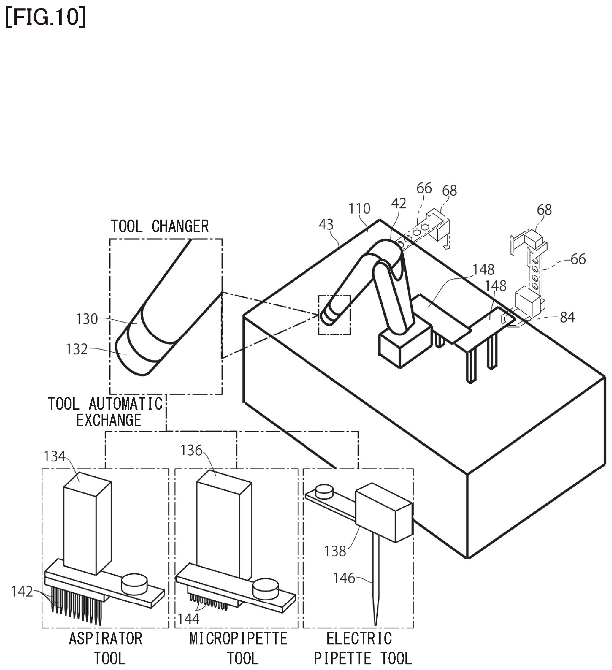

Further, although details will be described later, various operation devices (operation apparatuses) such as an aspirator tool 134, a micropipette tool 136, and a pipettor tool 138 illustrated in FIG. 10, an air gripper 68 illustrated in (a) of FIG. 7, and the like are attached to the respective distal end portions of the culture medium exchange robot 42 and the transfer robot 40. Then, the culture medium exchange robot 42 and the transfer robot 40 perform predetermined operations using various operation devices within respective movable ranges.

### <Another description 1 regarding layout of portions (arrangement in plurality of quadrants)>

The layout of the portions (incubator portion 12, image inspection portion 14, stocker supply portion 16, product removal portion 18, transfer robot portion 20, and culture medium exchange portion 22) will also be described as follows in addition to the description that the portions are disposed so as to surround the transfer robot portion 20 as described above.

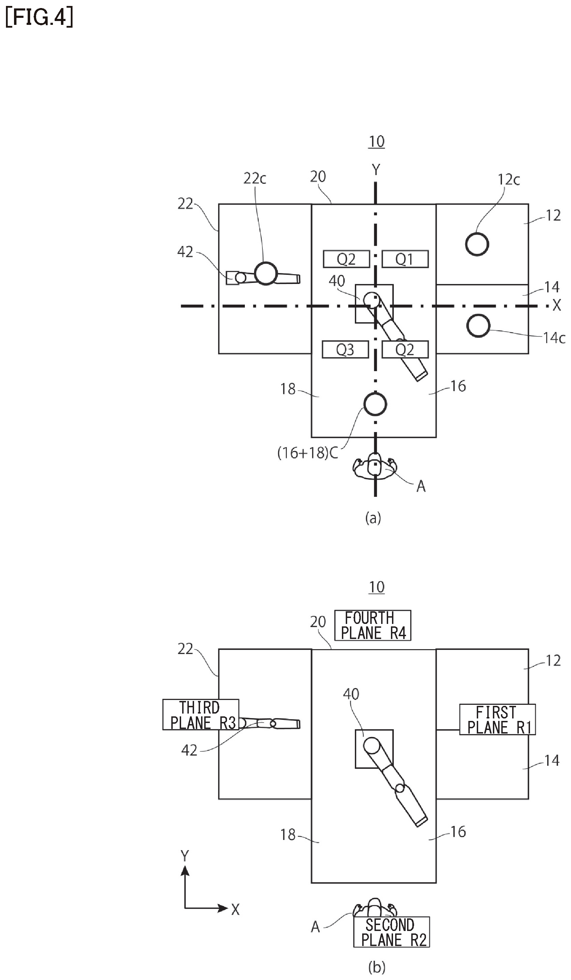

For example, as illustrated in (a) of FIG. 4, XY coordinates are planarly set with the position of the rotation center of the transfer robot 40 as an origin, and a space whose position is defined by the XY coordinates is divided into a first quadrant Q1 to a fourth quadrant Q4. Then, central portions 12C, 14C, (16+18)C, and 22C of the portions are disposed in at least three quadrants (here, the first quadrant Q1, the boundary portion between the second quadrant Q2 and the third quadrant, and the fourth quadrant Q4) among the first quadrant Q1 to the fourth quadrant Q4.

Here, (16+18)C in (a) of FIG. 4 indicates a central portion between the stocker supply portion 16 and the product removal portion 18. Further, in (a) of FIG. 4, the central portion (20C) of the transfer robot portion 20 is not illustrated.

In the example of (a) in FIG. 4, the central portion 12C of the incubator portion 12 is disposed in the first quadrant Q1, and the central portion 14C of the image inspection portion 14 is disposed in the second quadrant Q2. Furthermore, the central portion (16+18)C where the stocker supply portion 16 and the product removal portion 18 are combined is disposed between the second quadrant Q2 and the third quadrant Q3 (on the Y-axis). Furthermore, the central portion 22C of the culture medium exchange portion 22 is disposed in the fourth quadrant Q4.

Note that, although not illustrated, the central portion (16+18)C combining the stocker supply portion 16 and the product removal portion 18 may also be defined as being disposed across the second quadrant Q2 and the third quadrant Q3. Further, it may also be defined that the central portion (16C) of the stocker supply portion 16 is disposed in the second quadrant Q2 and the central portion (18C) of the product removal portion 18 is disposed in the third quadrant. Furthermore, the central portion 20C of the transfer robot portion 20 is disposed at the origin of the XY coordinates. Thus, the central portion 20C of the transfer robot portion 20 may be defined as being disposed across the first quadrant Q1 to the fourth quadrant Q4.

The description of the layout based on such a quadrant may be expressed as follows, for example. That is, in the cell culture apparatus 10, in an orthogonal coordinate system centered on the second operation portion (such as the transfer robot 40) in plan view, the respective central portions (central portions 22C, (16+18)C, 12C, 14C, and the like) in the first space (the internal space related to the culture medium exchange portion 22), the supply and discharge portion (such as the stocker supply portion 16), the cell culture portion (such as the incubator portion 12), and the inspection portion (such as the image inspection portion 14) are disposed separately in at least three quadrants (first quadrant Q1, second quadrant Q2 (or third quadrant), fourth quadrant Q4, and the like).

### <Another description 2 regarding layout of portions (arrangement on plurality of planes)>

(b) of FIG. 4 illustrates another description example of the layout of the portions. In the example of (b) of FIG. 4, XY coordinates are planarly set with the position of the rotation center of the transfer robot 40 as an origin, and a space whose position is defined by the XY coordinates is divided into a first plane R1 to a fourth plane R4 depending on the position and the direction. The portions are disposed on three planes (here, the first plane R1, the second plane R2, and the fourth plane R4) of the first plane R1 to the fourth plane R4.

In the example of (b) in FIG. 4, the incubator portion 12 and the image inspection portion 14 are disposed on the first plane R1, and the stocker supply portion 16 and the product removal portion 18 are disposed on the second plane R2. Furthermore, the culture medium exchange portion 22 is disposed on the third plane R3. The fourth plane located between the first plane R1 and the third plane R3 and facing the second plane R2 is an open plane on which the portions are not disposed. The open plane (fourth plane R4) is a plane on which the opening and closing shutter portion 36 (FIG. 5) of the transfer robot portion 20 is disposed. Note that in the example of (b) of FIG. 4, the portions are disposed on three planes (three planes) but may be collectively disposed on two planes (two planes).

### <Basic configuration of portions>

FIG. 5 illustrates a basic configuration of the portions (incubator portion 12, image inspection portion 14, stocker supply portion 16, product removal portion 18, transfer robot portion 20, and culture medium exchange portion 22). Hereinafter, the basic configuration of the portions will be described. Prior to the description of each portion, the well plate 52 movable between the portions and the stocker 54 on which the well plate 52 is placed will be described.

### <Well plate 52 and stocker 54>

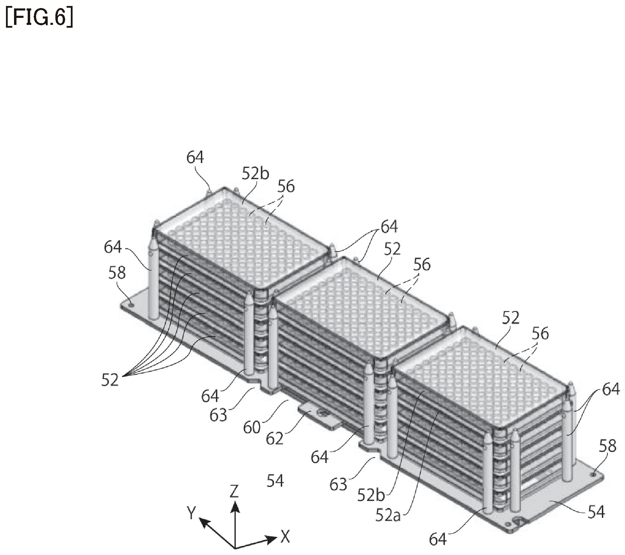

FIG. 6 illustrates the well plates 52 and the stocker 54. Each of the well plates 52 used in the present embodiment has a common shape and structure. In each of the well plates 52, a large number of recessed wells 56 for accommodating a culture solution or the like are formed. In the present embodiment, 96 (= 8 × 12) wells 56 are formed in a matrix shape in each well plate 52.

The well plate 52 is configured by covering a well plate body 52a with a lid 52b. The well plate body 52a and the lid 52b are formed in a rectangular container shape with one side open. By placing the lid 52b over the well plate body 52a, the face of the well plate body 52a on which the wells 56 are open is covered. The well plate body 52a and the lid 52b are formed of a transparent synthetic resin material. Then, the inside of the well 56 can be visually recognized from both the lid 52b side and the back side of the well plate body 52a (side opposite to the side covered with the lid 52b).

The lid 52b merely covers the well plate body 52a, and the lid 52b is not fixed to the well plate body 52a. Thus, for example, by lifting (pulling up) the lid 52b in the positive direction (upward direction) in the Z direction in FIG. 6, the lid 52b is separated from the well plate body 52a.

The well plate 52 is placed on the rectangular plate-shaped stocker 54. A maximum of 15 (= 3 × 5) well plates 52 are placed on one stocker 54. The well plate 52 may also be counted as "sheets" or "stages". In the example of FIG. 6, the well plates 52 are stacked in the vertical direction (Z direction) as a set of five well plates. Furthermore, three sets of well plates 52 are arranged so that the longitudinal direction (Y direction in FIG. 6) of the well plates coincides with the longitudinal direction (Y direction in FIG. 6) of the stocker 54.

The stocker 54 is formed using, for example, a material such as stainless steel. A hole 58, a rectangular notch 60, a protrusion 62, a V-shaped notch 63, and the like are formed at an edge of the stocker 54. The rectangular notch 60 is located at the central portion of each edge (each edge facing in the transverse direction (width direction)) extending in the longitudinal direction of the stocker 54.

The protrusion 62 is located at the central portion of the rectangular notch 60 in the length direction. The V-shaped notches 63 are disposed with the rectangular notch 60 interposed therebetween in the length direction. The V-shaped notches 63 have a shape that narrows toward the back (the back in the transverse direction (width direction) of the stocker 54).

Furthermore, a large number (16 in this embodiment) of surrounding pins 64 are attached to a peripheral edge of the stocker 54. These surrounding pins 64 protrude substantially perpendicularly from the plate surface on which the well plates 52 are placed. Among these, 12 (= 6 × 2) surrounding pins 64 are disposed to face the well plates 52 arranged in the longitudinal direction of the stocker 54 at a portion in the vicinity of the four corner portions with a predetermined spacing (spacing of about several millimeters).

Most of the side surfaces (side surfaces constituting the longitudinal direction) of the three sets of well plates 52 are exposed without being hidden by the surrounding pins 64. By disposing the surrounding pins 64 with a spacing in the longitudinal direction of the stocker 54 (and the well plates 52) in this manner, a space for allowing a grip portion 76 (FIG. 7) of the transfer robot 40 to enter is secured as described later. Further, 4 (= 2 × 2) of the 12 surrounding pins 64 are disposed to face the two corner portions of each of the two sets of well plates 52 at both ends with a predetermined spacing (spacing of about several millimeters) therebetween.

The tips (upper ends in FIG. 6) of the surrounding pins 64 are formed to be sharp. When the well plate 52 is placed on the stocker 54, the well plate 52 is placed on a portion surrounded by the surrounding pins 64 from above in a state of being handled by an air gripper 68 (described later) having a grip portion 76 (FIG. 7). Since the tips of the surrounding pins 64 (the upper ends in FIG. 6) are sharp, the tips of the surrounding pins 64 hardly interfere with the well plate 52 when the well plate 52 is placed on the stocker 54. Note that it is not essential to provide the surrounding pins 64, and the surrounding pins 64 may be omitted.

### <Transfer robot 40>

The transfer of the stocker 54 is performed by the transfer robot 40. The transfer robot 40 is a vertical articulated robot as described above. For example, the transfer robot 40 has a feeding accuracy of about ±0.02 mm. Main functions of the transfer robot 40 are gripping (nipping) and transferring the stocker 54 and the well plate 52. The transfer robot 40 can grip and transfer the stocker 54 on which the well plate 52 is placed or can grip and transfer only the stocker 54 without placing the well plate 52.

Further, the transfer robot 40 can also grip and transfer only the well plate 52. Furthermore, the transfer robot 40 can also grip and transfer only the lid 52b in order to separate the lid 52b of the well plate 52 from the well plate body 52a or cover the well plate body 52a. Gripping and transferring the stocker 54, the well plate 52, and the lid 52b by the transfer robot 40 are performed via the following mechanism.

An arm plate 66 (FIG. 10) reduced in weight by lightening is attached to the distal end portion of the transfer robot 40, and an air gripper 68 as illustrated in (a) of FIG. 7 is attached to one end portion of the arm plate 66. Here, the arm plate 66 and the air gripper 68 are used at various positions and orientations depending on the process, but FIG. 10 illustrates the arm plate 66 and the like in a plurality of processes at the same time. In FIG. 10, in order to illustrate that the arm plate 66 is virtually drawn, lead lines by two-dot chain lines (virtual lines) are attached to the arm plate 66.

The air gripper 68 includes one air cylinder 70 and two movable bodies 72. A plurality of pipe joints 74 is attached to the air cylinder 70, and although not illustrated, for example, a flexible resin tube is connected to each pipe joint 74. In the air cylinder 70, high pressure air supplied from an air source (air compressor or the like) via a resin tube (not illustrated) is introduced and discharged. Then, the two movable bodies 72 perform an operation of expanding the spacing and an operation of narrowing the spacing in accordance with the introduction and discharge of the high-pressure air.

A T-shaped grip portion 76 is fixed to each of the movable bodies 72. A metal plate-shaped ledge (claw) portion 78 is attached to a distal end of each grip portion 76. Each of the ledge portions 78 is provided with two resin blocks 80 and two locking pins 82. The resin blocks 80 are made of a synthetic resin and partially cover edges of two grip portions 76 facing each other.

The locking pins 82 are disposed at respective ends of the ledge portions 78 in the longitudinal direction. The locking pins 82 are each formed in a stepped columnar shape and protrude substantially perpendicularly from the plate surface of the grip portions 76. The locking pins 82 each have a relatively small diameter portion on a proximal end side (side close to the ledge portion 78) and a relatively large diameter portion on a distal end side (side far from the ledge portion 78).

(b) of FIG. 7 illustrates a situation in which the grip portion 76 is locked to the stocker 54. When gripping the stocker 54, the air cylinder 70 approaches the central portion of the stocker 54 from above, and the grip portion 76 faces an edge of the well plate 52 in a state of straddling the well plate 52 and the stocker 54 in the width direction. In (b) of FIG. 7, only one of the grip portions 76 is illustrated.

As indicated by arrow B in (b) of FIG. 7, when the two grip portions 76 (only one is illustrated) are brought close to each other, the ledge portion 78 enters below the protrusion 62 formed at the edge of the stocker 54. At this time, the two resin blocks 80 of the ledge portion 78 reach the position where the protrusion 62 is placed therebetween and face the side surface of the well plate 52 at the lowermost stage.

Further, the locking pins 82 approach the respective V-shaped notches 63 formed at the edge of the stocker 54. Then, a portion on the small-diameter proximal end side of each locking pin 82 enters the V-shaped notch 63, and a portion on the large-diameter distal end side of the locking pin 82 partially overlaps the edge of the V-shaped notch 63.

With such a closing operation of the grip portion 76, the four locking pins 82 are guided by obliquely formed edges of the corresponding V-shaped notches 63. The four locking pins 82 enter deeply into the respective V-shaped notches 63 and are locked to the stocker 54. As a result, the stocker 54 is positioned with respect to the ledge portion 78 and gripped by the grip portion 76.

When the distal end portion of the transfer robot 40 moves upward in a state where the grip portion 76 grips the stocker 54, the air cylinder 70 is also integrally displaced. The ledge portion 78 of the grip portion 76 comes into contact with the protrusion 62 of the stocker 54 and other portions from below and ascends, and the stocker 54 is lifted.

At this time, a force with which the grip portion 76 grips the stocker 54 is relatively small, and a force required for the ledge portion 78 to support the stocker 54 is larger and dominant than the force with which the grip portion 76 grips the stocker 54. Then, the transfer robot 40 transfers the stocker 54 and the well plate 52 placed on the stocker 54 to a target position depending on the process while maintaining the level of the stocker 54.

The presence or absence, the number of stages, and the arrangement of the well plates 52 placed on the stocker 54 differ depending on the process. The air gripper 68 including the air cylinder 70, the grip portion 76, and the like supports and transfers the stocker 54 in a situation where the total weight and weight balance of the object to be gripped are different. The stocker 54 and the well plate 52 are each standardized by adopting a common size, structure, and the like. Thus, it is possible to transfer a large number of stockers 54 and well plates 52 using a single (one type of) air gripper 68.

When the transfer robot 40 transfers the well plate 52 with the lid 52b alone, the well plate body 52a is gripped in a state of being covered with the lid 52b, and the well plate body 52a is gripped together with the lid 52b. Further, when the transfer robot 40 transfers the lid 52b alone, only the lid 52b is gripped and lifted, and is removed from the well plate body 52a.

When the air gripper 68 grips the well plate 52 or the lid 52b, an elevation position (position in the vertical direction (Z direction)) of the air gripper 68 and the spacing between the grip portions 76 (and the ledge portions 78) are different from those in the case of gripping the stocker 54. Further, when the well plate 52 is gripped and when the lid 52b is gripped, the elevation position of the air gripper 68 and the spacing between the grip portions 76 are different.

When the air gripper 68 grips the well plate 52 or the lid 52b, the elevation position of the air gripper 68 is higher than the case of gripping the stocker 54, and the spacing between the grip portions 76 is narrower than the case of gripping the stocker 54. Further, in a case where the air gripper 68 grips the lid 52b, the elevation position of the air gripper 68 becomes higher and the spacing between the grip portions 76 becomes wider than in a case where the well plate 52 (the well plate body 52a) is gripped.

When the air gripper 68 grips the well plate 52 or the lid 52b, the two ledge portions 78 of the grip portion 76 gradually approach, and the four (2 × 2) resin blocks 80 come into contact with the side surface of the well plate body 52a or the lid 52b. Then, the well plate body 52a and the lid 52b are gripped using a frictional force accompanying pressurization from the side surface without directly bringing the ledge portion 78 into contact with the well plate body 52a or the lid 52b. Thus, the well plate body 52a and the lid 52b made of a transparent resin can be prevented from being damaged by the metal ledge portion 78.

The transfer robot 40 can also rotate about each of orthogonal axes (XYZ axes). Thus, the transfer robot 40 may rotate the well plate 52 and the stocker 54 at an angle of 90 degrees or the like while maintaining the horizontal state at the time of transferring the well plate 52 and the stocker 54 depending on the process.

Note that in the present embodiment, a fork portion 84 (FIG. 10, two long claw portions) is formed at the other end portion of the arm plate 66 (FIG. 10) to which the air gripper 68 is attached at one end. The fork portion 84 can hold reservoirs 120 one by one by supporting both edges of the reservoirs 120 used in the culture medium exchange portion 22 as described later. That is, the transfer robot 40 can transfer four types of objects including the stocker 54, the well plate 52, the lid 52b, and the reservoir 120.

### <Path of transfer by transfer robot 40>

The transfer robot 40 transfers the stocker 54 on which the well plate 52 is placed, the well plate 52, and the lid 52b, for example, between the stocker supply portion 16 and the incubator portion 12, between the incubator portion 12 and the image inspection portion 14, between the incubator portion 12 and the culture medium exchange portion 22, between the incubator portion 12 and the product removal portion 18, and the like depending on the process. In FIG. 5, as an example, a path from the stocker supply portion 16 to the incubator portion 12 and a path from the incubator portion 12 to the product removal portion 18 are schematically indicated by arrows C1 and C2.

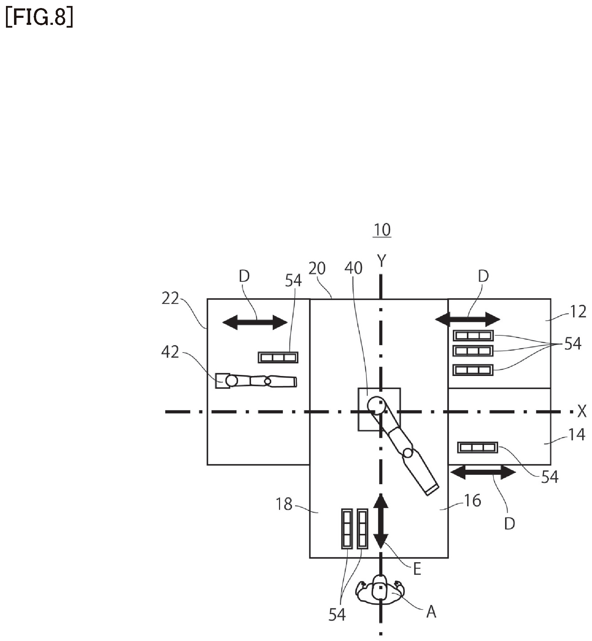

As indicated by thick arrows D and E in FIG. 8, in the four sections of the incubator portion 12, the image inspection portion 14, the stocker supply portion 16 (stocker supply and removal portion), and the culture medium exchange portion 22, the direction (orientation) of the stocker 54 to be placed is set so that the longitudinal direction of the stocker 54 is parallel to the X-axis or the Y-axis. By disposing the stocker 54 in such a direction, the transfer robot 40 can dispose the stocker 54 so as to push the stocker 54 in the longitudinal direction. Therefore, the stocker 54 can be prevented from interfering with a peripheral portion, and the plurality of stockers 54 can be easily disposed.

Further, in a situation where the stocker 54 is not gripped, the distal end portion of the transfer robot 40 does not need to be greatly raised so that the air gripper 68 can be avoided from interfering with the well plate 52 and the like. Therefore, the movement path of the transfer robot 40 can be simplified.

### <Incubator portion 12>

The stocker 54 on which the well plate 52 is placed is taken in and out of the incubator portion 12 by the transfer robot 40. As illustrated in FIGS. 2 and 5, the incubator portion 12 is provided with the incubator 32 capable of controlling the internal temperature, and the incubator 32 is provided with an automatic door 86. The automatic door 86 is a one-side hinge type door and turns (rotationally displaces) in the horizontal direction. When the stocker 54 is taken in and out, for example, the automatic door 86 is opened and closed under the control of the control portion 46 (FIG. 5). FIG. 2 illustrates a state in which the automatic door 86 is opened. Further, in FIG. 5, a state in which automatic door 86 is opened is indicated by a broken line. Furthermore, the door 37 illustrated in the upper left portion in FIG. 5 is also used for entry and exit of the operator A or the like.

### <Image inspection portion 14>

(a) and (b) of FIG. 9 illustrate an outline of the image inspection portion 14. The image inspection portion 14 includes an image inspection apparatus 34, and the image inspection apparatus 34 includes a camera 90. The camera 90 is disposed at a constant height and can capture an image downward. Note that, although not illustrated, the image inspection apparatus 34 also includes a camera that captures an image upward from below.

The camera 90 moves in the XY plane by two-axis linear guide device 92. The linear guide device 92 is configured by combining electric actuators 94, 96 corresponding to the respective axes. In the examples of (a) and (b) of FIG. 9, one electric actuator 94 is for the X-axis, and the other electric actuator 96 is for the Y-axis. As illustrated in (b) of FIG. 9, the X-axis electric actuator 94 is installed on the Y-axis electric actuator 96 via a support column 98.

Note that the X-axis and the Y-axis of the image inspection apparatus 34 illustrated in (a) and (b) of FIG. 9 are different from the directions of the X-axis and the Y-axis of the cell culture apparatus 10 illustrated in FIGS. 1 to 3, 8, and the like. The X-axis direction of the image inspection apparatus 34 coincides with a Y-axis direction of the cell culture apparatus 10, and the Y-axis direction of the image inspection apparatus 34 coincides with the X-axis direction of the cell culture apparatus 10. Further, the example of (a) and (b) of FIG. 9 is different from the examples of FIGS. 1 to 3, 8, and the like in the arrangement of the linear guide device 92. For example, the orientation of the image inspection apparatus 34 of the example of (a) and (b) of FIG. 9 can be rotated to match the orientation of FIGS. 1 to 3, 8, and the like.

The camera 90 constitutes an image inspection system. Although not illustrated, the image inspection system is constructed by combining the camera 90 and industrial computer equipment (industrial PC). The image inspection system has a cell pass/fail determination function. An inspection related to the culture state of the cell is performed using a captured image of the camera 90, and inspection data obtained by the inspection is stored in an external storage such as a hard disk drive (HDD) or a solid state drive (SSD). Further, the inspection data is stored in association with information related to the history of cell culture.

The transfer robot 40 transfers the stocker 54 taken out from the incubator portion 12 to the image inspection portion 14. In the image inspection portion 14, the stocker 54 is placed in a predetermined pre-inspection placement region 100 with the longitudinal direction thereof oriented in the Y-axis direction (X-axis direction of the cell culture apparatus 10). The well plate 52 is transferred from the stocker 54 to an inspection region 102 below the camera 90 and placed therein as indicated by arrow F in (b) of FIG. 9.

As described above, the well plate body 52a and the lid 52b constituting the well plate 52 are transparent, and the inspection in the inspection region 102 is performed through the lid 52b while covering the well plate body 52a with the lid 52b. The well plate 52 (for example, the well plate body 52a) is provided with an identification information display portion (not illustrated), and an image of the identification information display portion is captured in the inspection region 102.

Information (identification information) capable of individually identifying the well plate 52 is displayed on the identification information display portion. The identification information can be displayed by attaching a label on which a barcode or a two-dimensional matrix code is drawn, or by engraving a symbol for identification. Further, reading of the identification information is performed while illuminating the identification information display portion with a lighting device (not illustrated).

When the image-capturing or inspection of the well plate 52 placed in the inspection region 102 by the camera 90 is completed, the well plate 52 is returned to the pre-inspection placement region 100 side as indicated by arrow G. The temporary placement region 104 is provided adjacent to the pre-inspection placement region 100, and the well plate 52 after the inspection is placed in the temporary placement region 104 by the transfer robot 40. In the temporary placement region 104, the stocker 54 is placed in advance, and the well plate 52 after the inspection is placed on the stocker 54 of the temporary placement region 104. The position of the temporary placement region 104 is a position between the pre-inspection placement region 100 and the inspection region 102 and close to the pre-inspection placement region 100.

The subsequent well plate 52 is similarly inspected, and the well plate 52 after the inspection is sequentially stacked on the stocker 54 of the temporary placement region 104. When the number of stacked well plates 52 reaches a predetermined number (five in this embodiment), the subsequent (sixth and subsequent) well plates 52, which have been inspected, are stacked at adjacent positions in the longitudinal direction on the stocker 54 up to 5.

When the number of well plates 52 in the temporary placement region 104 reaches 15 with five well plates 52 stacked at each of three locations, the stocker 54 on which these well plates 52 are placed is gripped and transferred by the transfer robot 40 and accommodated in the incubator portion 12.

For example, the inspection of the cells in the image inspection portion 14 is performed by simultaneously imaging all (96 in this embodiment) wells 56 of the well plate body 52a from above, performing image recognition of the cells in each well 56, and performing predetermined image processing. A cell having a size or a shape that has not reached a predetermined inspection criterion is recorded as a failure, and the inspection result is notified via, for example, a display device 106 (FIG. 1) provided outside the image inspection portion 14. Note that the contents of the inspection, the inspection criteria, the method of notifying the inspection result, and the like are not limited to those described here and may be variously changed or added.

Note that artificial intelligence (AI) can be used for the inspection. For example, it is possible to cause the control portion 46 to learn in advance the relationship between the conditions related to the size and shape of the imaged cell and the quality (pass/fail) of the cell, and the control portion 46 determines a cell whose correlation with the learning result of a pass does not fall within a predetermined amount as a failure and notifies the failure. Further, it is also possible that the control portion 46 is caused to learn the growth record of the cell in advance, and the control portion 46 predicts the degree of subsequent growth from the previous growth record for the same sample. The contents of the inspection using AI can also be changed in various ways.

Further, although not illustrated, it is possible to install a sterilization lamp (or germicidal lamp) by, for example, an ultraviolet (UV) light source (ultraviolet LED or the like) at an appropriate location in the image inspection portion 14 and perform sterilization (or germicidal treatment) in the image inspection portion 14. The installation of such a sterilization portion (or germicidal portion) is effective for preventing the propagation of bacteria and keeping the internal environment clean, for example, in a case where a culture solution drips (dripping), in a case where a person comes in contact, or the like.

Sterilization (or germicidal treatment) by light irradiation can be performed locally on a place where dripping or the like is likely to occur. In this manner, the ultraviolet light source can be appropriately disposed by selecting an installation place. For example, it is possible to appropriately adjust the relationship between the position of the synthetic resin part that is easily deteriorated by the ultraviolet ray and the position of the ultraviolet light source so that the ultraviolet ray does not hit the synthetic resin part, and to perform arrangement thereof. Furthermore, the ultraviolet light source may be disposed at a movable portion of a part (for example, the arm plate 66, the air gripper 68, and the like illustrated in FIG. 10) of the transfer robot 40.

Further, regarding the timing of sterilization (or germicidal treatment) by light irradiation, for example, it is possible to perform light irradiation in a time period (so-called idle time, standby state, and the like) in which various operations by the transfer robot 40 are not performed under the control of the control portion 46.

### <Culture medium exchange portion 22>

FIG. 5 illustrates an outline of the culture medium exchange portion 22. In the culture medium exchange portion 22, in addition to the above-described culture medium exchange robot 42 installed on a worktable 110, an aspirator tool holder 112, disposal boxes 114 and 115, a micropipette tool holder 116, a pipettor tool holder 118, a reservoir 120, a pipette bit 122, an aspiration bit 124, a culture medium tank installation portion 126, and the like are provided so as to surround the periphery of the culture medium exchange robot 42.

Among them, as illustrated in FIG. 10, a tool changer 130 is attached to the distal end portion of the culture medium exchange robot 42. A tool adapter 132 is provided at a distal end of the tool changer 130, and any operation device such as the aspirator tool 134, the micropipette tool 136, or the pipettor tool 138 can be attached to the tool adapter 132.

Note that FIGS. 5 and 10 each illustrate an example of arrangement of each device, and the arrangement of each device does not necessarily coincide between FIGS. 5 and 10.

In operation devices such as the aspirator tool 134, the micropipette tool 136, and the pipettor tool 138 illustrated in FIG. 10, the operation devices have a common connection structure for the tool adapter 132. Thus, it is possible to interchangeably connect various operation devices (134, 136, 138, and the like) to one tool adapter 132. Further, the tool adapter 132 is provided with a fall prevention function for preventing fall of an operation device (134, 136, 138, and the like).

Among the operation devices (134, 136, 138, and the like), the aspirator tool 134 includes 12 distal end bits (needles) 142 disposed parallel to each other and can simultaneously aspirate the culture medium (culture solution) in the 12 wells 56 arranged in the longitudinal direction via the distal end bits 142.

The micropipette tool 136 also includes 12 needles 144 disposed in parallel to each other and can simultaneously discharge a culture medium (culture solution) to 12 wells 56 via the needles 144.

The pipettor tool 138 draws up the culture solution from a culture solution bottle (not illustrated) and injects the culture solution into the reservoir 120 before the micropipette tool 136 discharges the culture medium. The culture solution in the reservoir 120 is aspirated by the micropipette tool 136 and discharged to the well 56 of the well plate body 52a. The pipettor tool 138 is of a rechargeable type.

In the aspirator tool holder 112, the micropipette tool holder 116, and the pipettor tool holder 118, the aspirator tool 134, the micropipette tool 136, and the pipettor tool 138 in a state (non-used state) not attached to the culture medium exchange robot 42 are placed, respectively.

The disposal box 114 is used for disposal of the distal end bit 142 of the aspirator tool 134. The distal end bit 142 is attached to the aspirator tool 134 so as to be automatically detachable, and the used distal end bit 142 is removed from the aspirator tool 134 and discarded to the disposal box 114.

The reservoir 120 is a portable container and is used not only for aspirating a culture solution but also for cleaning the distal end bit 142. At the time of cleaning the distal end bit 142, cleaning alcohol is injected into the reservoir 120, and the reservoir 120 is placed in a reservoir placement region 128 on the worktable 110. The cleaning alcohol may be injected using, for example, a suction pump (not illustrated) provided in the culture medium exchange portion 22.

The culture medium exchange robot 42 causes the tip of the distal end bit 142 in a state of being attached to the aspirator tool 134 to enter the cleaning alcohol in the reservoir 120, and aspirates, for example, the entire amount of the cleaning alcohol in the reservoir 120. As a result of aspiration, the cleaning alcohol is discharged to a waste liquid tank (not illustrated). Thereafter, the distal end bit 142 is automatically removed from the aspirator tool 134 and discarded in the disposal box 114.

In the culture medium exchange portion 22, as a countermeasure against dripping from various operation devices (134, 136, 138, and the like), a plurality of reception plates 148 formed of a stainless steel plate is installed at positions below flow lines (movement trajectories) of the various operation devices (134, 136, 138, and the like), for example, as illustrated in FIG. 10.

FIG. 11 schematically illustrates a configuration of the wall portion 22B of the culture medium exchange portion 22 and other elements. The culture medium exchange portion 22 includes an outer wall portion 22A, the inner wall portion 22B, and other elements. The outer wall portion 22A constitutes a part of the wall portion 10A in the cell culture apparatus 10. The inner wall portion 22B is located inside the wall portion 10A of cell culture apparatus 10 and divides an internal space of the wall portion 10A into a first space (internal space of culture medium exchange portion 22) and a second space (internal space other than culture medium exchange portion 22).

A manual opening and closing shutter 150 is provided on the outer wall portion 22A. The manual opening and closing shutter 150 is manually opened and closed, for example, when a consumable such as a culture solution is replenished. Supplementation of consumable items can also be performed by a transfer robot (not illustrated) installed outside the cell culture apparatus 10. An opening and closing direction of the manual opening and closing shutter 150 is a vertical direction as indicated by arrow H in FIG. 11. The manual opening and closing shutter 150 is locked (locked out) so as not to be opened or closed during drive of the culture medium exchange robot 42 or the like. The manual opening and closing shutter 150 is locked via an electromagnetic lock device under the control of the control portion 46, for example.

The automatic opening and closing shutter 152 is provided on the inner wall portion 22B. The automatic opening and closing shutter 152 is controlled for opening and closing under the control of the control portion 46, for example. Specifically, the automatic opening and closing shutter 152 is opened when the transfer robot 40 causes the arm plate 66 (FIG. 10) to enter the internal space (first space) of the culture medium exchange portion 22 from the second space. An opening and closing direction of the automatic opening and closing shutter 152 is a vertical direction as indicated by arrow J in FIG. 11.

Further, a sterilization lamp (or germicidal lamp) 154 by, for example, an ultraviolet (UV) light source (ultraviolet LED or the like) is installed in the culture medium exchange portion 22, and sterilization (or germicidal treatment) is performed in the culture medium exchange portion 22. Such a sterilization portion (or germicidal portion) is disposed at an appropriate position in the internal space (first space) so as to prevent propagation of bacteria and keep the internal environment clean, for example, when the culture solution drips (dripping) or when a person comes in contact therewith. Examples of the arrangement location of the sterilization lamp 154 include one to four sides among the four sides of the ceiling portion 24 facing the internal space (first space), an appropriate position on the worktable 110, and the like.

Sterilization (or germicidal treatment) by light irradiation can be performed locally on a place where dripping or the like is likely to occur. In this manner, the ultraviolet light source can be appropriately disposed by selecting an installation place. For example, it is possible to appropriately adjust the relationship between the position of the synthetic resin part that is easily deteriorated by the ultraviolet ray and the position of the ultraviolet light source so that the ultraviolet ray does not hit the synthetic resin part, and to perform arrangement thereof. Furthermore, the ultraviolet light source can be disposed on a movable portion of a part (for example, the tool changer 130, the aspirator tool 134, the micropipette tool 136, the pipettor tool 138, or the like) of the culture medium exchange robot 42.

Further, regarding the timing of sterilization (or germicidal treatment) by light irradiation, for example, it is possible to perform light irradiation in a time period (so-called idle time, standby state, and the like) in which various operations by the culture medium exchange robot 42 are not performed under the control of the control portion 46.

The transfer robot 40 transfers the stocker 54 taken out from the incubator portion 12 to the culture medium exchange portion 22 as described above, and the stocker is placed in a placement region 156 of the worktable 110. One well plate 52 is gripped by the transfer robot 40 from the stocker 54 and transferred to a culture medium exchange region 157. The lid 52b is removed by the transfer robot 40, and for example, one reservoir 120 located at the uppermost stage among the plurality of reservoirs 120 stacked in a reservoir preparation region 158 is supported and placed in the reservoir placement region 128. As described above, the reservoir 120 is supported and transferred by the transfer robot 40 using the arm plate 66 (FIG. 10).

Subsequently, the aspirator tool 134 attached to the culture medium exchange robot 42 aspirates the old medium from the well of the well plate body 52a. Thereafter, the aspirator tool 134 is automatically removed from the tool adapter 132 of the culture medium exchange robot 42 at the position of the aspirator tool holder 112.

The culture medium exchange robot 42 moves the tool adapter 132 at the distal end portion to the position of the pipettor tool holder 118, and the pipettor tool 138 is attached to the tool adapter 132. Furthermore, the culture medium exchange robot 42 moves the pipettor tool 138 to the reservoir placement region 128. Then, the culture medium is electrically injected into the reservoir 120 placed in the reservoir placement region 128 by the pipettor tool 138.

Furthermore, in the position of the pipettor tool holder 118, the pipettor tool 138 is automatically removed, and the culture medium exchange robot 42 moves the tool adapter 132 to the position of the micropipette tool holder 116. The micropipette tool 136 is automatically attached to the tool adapter 132 at the position of the micropipette tool holder 116. Then, the culture medium exchange robot 42 moves the micropipette tool 136 to the position of the reservoir 120, and the culture solution is aspirated from the reservoir 120 into the needle 144 of the micropipette tool 136.

The culture medium exchange robot 42 transfers the micropipette tool 136 to the position of the well plate body 52a to be subjected to the culture medium exchange, and the micropipette tool 136 injects the culture medium into the well 56 immediately below. For example, when the exchange of the culture medium for the 15 well plates 52 in one stocker 54 is completed, the stocker 54 on which the well plates (15 well plates) 52 whose culture medium has been exchanged are placed is returned to the incubator portion 12 by the transfer robot 40.

Note that when the aspirator tool 134 is used, the aspirator tool 134 (distal end bit 142) is inclined at a predetermined angle with respect to the well 56 to aspirate the culture medium. According to the findings of the inventors and the like, in this manner, when the culture medium is aspirated, the cells are significantly less likely to be aspirated together with the culture medium from a part (for example, about 1 to 3) of the 96 wells 56. Although the verification of the factor is not completely completed, from the findings of the inventors and the like, it is considered that aspirating the culture medium from an eccentric position with respect to the center of the well 56 is one factor of improvement.

Also when the micropipette tool 136 is used, the micropipette tool 136 (needle 144) is inclined or eccentric with respect to the well 56 at a predetermined angle, whereby the culture solution can be injected after being applied to the inner wall of the well 56, and the influence of the injection on the cells can be prevented as much as possible.

### <Safety improvement measures>

In the cell culture apparatus 10, for example, various processes necessary for cell culture are automated, and the transfer robot 40 performs various operations. For example, it is difficult for a person to predict the operation of the transfer robot 40. Thus, it is desirable that the cell culture apparatus 10 adopt safety improvement measures for enabling a person to safely enter the second space (a portion other than the culture medium exchange portion 22).

As safety improvement measures, various methods can be adopted. For example, it is conceivable that a human detection sensor 159 (FIG. 5) capable of detecting that a person is present in the cell culture apparatus 10 is provided, and the transfer robot 40 is not caused to perform operations such as gripping and transfer while the human detection sensor 159 detects a person under the control of the control portion 46. As the human detection sensor 159, for example, various sensors such as a load sensor (weight sensor), a pressure sensor, an optical sensor (including a laser sensor), an infrared sensor, a microwave sensor, an ultrasonic sensor, a camera sensor (image recognition sensor), and an acoustic sensor can be used.

Further, when a person enters the cell culture apparatus 10, the opening and closing shutter portion 36 (FIG. 5) is opened, but when a door opening sensor (not illustrated) detects opening of the opening and closing shutter portion 36, the control portion 46 can stop the operation of the transfer robot 40. Furthermore, when an operation (button operation, unlocking operation, and the like) for opening the opening and closing shutter portion 36 is performed, the operation of the transfer robot 40 can be stopped. Examples of the unlocking operation include input of a security key (number, symbol, or the like) with a key input device such as a number, insertion of a key into a key hole, and operation (rotation operation or the like) of a key inserted into a key hole.

Further, it is also possible that a person wears a device (wireless transmitter, identification information tag (RFID tag), and the like) capable of transmitting a wireless radio wave on work clothes, and when the transmitted wireless radio wave is detected by a reception portion provided in the cell culture apparatus 10, the operation of the transfer robot 40 is stopped. Furthermore, it is also possible to enable opening of the opening and closing shutter portion 36 only in a predetermined fixed time period. In this case, control using the human detection sensor 159 and a door opening sensor (not illustrated) can be omitted.

Note that when stopping the operation of the transfer robot 40, the operation of the movable portion in the entire cell culture apparatus 10 may be stopped including the culture medium exchange robot 42. Furthermore, it is also possible to stop only the transfer robot 40 and the culture medium exchange robot 42.

Further, since the culture medium exchange robot 42 is partitioned by the wall portion 22B of the culture medium exchange portion 22 or the like, it is considered that safety is ensured. Furthermore, in the cell culture apparatus 10 of the present embodiment, a person does not enter the culture medium exchange portion 22 in the process. Therefore, even if the operation of the transfer robot 40 is stopped, the culture medium exchange robot 42 is not stopped, and the operation related to the culture medium exchange can be performed.

### <Main merit of cell culture apparatus 10>

The cell culture apparatus 10 of the present embodiment includes the control portion 46 that sets the movable range in a case where the first operation portion (culture medium exchange robot 42) performs the operation related to the culture medium exchange as the first space (the internal space of the culture medium exchange portion 22), and sets the movable range in a case where the second operation portion (transfer robot 40) moves the well plate 52 as the second space and the first space.

Therefore, the movable range of the first operation portion (culture medium exchange robot 42) and the movable range of the second operation portion (transfer robot 40) can overlap each other, and space efficiency in the cell culture apparatus 10 can be optimized. Then, space saving can be achieved, and the compact cell culture apparatus 10 can be provided.

Further, since the first operation portion capable of executing the operation related to the exchange of the culture medium is selectively disposed in the first space having a relatively high air cleanliness (high cleanliness), the second operation portion capable of executing the moving operation of the cell culture container is disposed in the second space having a relatively low air cleanliness, the movable range in a case where the first operation portion performs the operation related to the exchange of the culture medium is the first space, and the movable ranges in a case where the second operation portion moves the cell culture container are the second space and the first space, the operation portions having different functions can be operated under a more optimized environment.

Further, the portions (incubator portion 12, image inspection portion 14, stocker supply portion 16, product removal portion 18, and culture medium exchange portion 22) are disposed around the transfer robot portion 20 with the transfer robot portion 20 as the center. Therefore, the space efficiency can also be optimized by this. Then, space saving can be achieved, and the compact cell culture apparatus 10 can be provided.

Further, in the culture medium exchange portion 22, since various regions (aspirator tool holder 112, disposal boxes 114, 115, micropipette tool holder 116, pipettor tool holder 118, reservoir 120, pipette bit 122, aspiration bit 124, culture medium tank installation portion 126, and the like) necessary for the operation of culture medium exchange are disposed so as to surround the culture medium exchange robot 42, the space efficiency can be optimized. Then, regarding the arrangement of the region necessary for the operation of culture medium exchange, space saving can be achieved, and the small culture medium exchange portion 22 can be provided.

Further, the well plate 52 is transferred not only to the space (first space) having a relatively high air cleanliness (high degree of cleanliness) but also to the second space having a relatively low air cleanliness. Since the lid 52b is provided, it is possible to prevent dust on the well 56 and to keep the well 56 clean.

Further, most of the processes of storing the well plate 52 and the stocker 54 in the incubator 32, taking out the well plate 52 and the stocker 54 from the incubator 32, the cell inspection in the image inspection portion 14, and the exchange of the culture medium in the culture medium exchange portion 22 can be automated, and work required for cell culture can be reduced. Note that the inspection of cells is not limited to the image inspection described in the present embodiment, and various inspection methods can be adopted.

Further, regarding the transfer robot 40, since different types of gripping targets (and transfer targets) such as the stocker 54, the well plate 52, and the lid 52b are gripped (and transferred) by a single (one type) air gripper 68, it is unnecessary to exchange the air gripper 68 for each gripping target (and transfer target). Therefore, the transfer robot 40 can perform multiple functions. Furthermore, the gripping (and transfer) of the gripping target (and the transfer target) can be easily automated.

Furthermore, a plurality of sets of well plates 52 is arranged in the stocker 54, each set including a plurality (five in this embodiment) of the well plates. Therefore, it is possible to simultaneously transfer a large number of well plates 52.

Further, in the culture medium exchange robot 42, the tool changer 130 is equipped with the tool adapter 132, and various operation devices (aspirator tool 134, micropipette tool 136, pipettor tool 138, or the like) are interchangeably connected to one tool adapter 132. Therefore, the culture medium exchange robot 42 can perform multiple functions. Furthermore, the operation device can be easily exchanged, and the exchange of the operation device can be easily automated.

Further, since the transfer robot 40 stops when entry of a person is detected, it is possible to operate the small-sized cell culture apparatus more safely.

### <Modification related to layout>

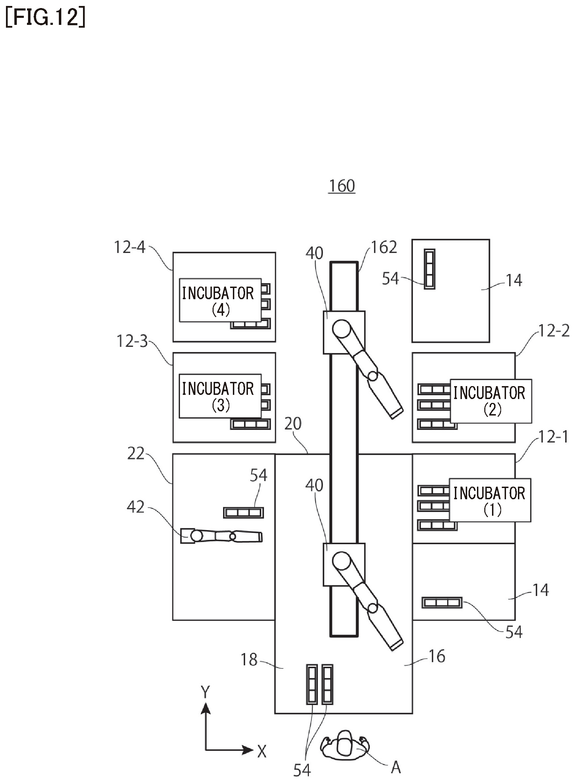

Note that the arrangement of the portions (incubator portion 12, image inspection portion 14, stocker supply portion 16, product removal portion 18, transfer robot portion 20, and culture medium exchange portion 22) is not limited to that illustrated in FIG. 1 and the like and may be variously changed. FIG. 12 illustrates a layout change example. A cell culture apparatus 160 in the example of FIG. 12 includes four incubator portions 12-1 to 12-4, and three incubator portions 12-2 to 12-4 are added as compared with the example of FIG. 1 and the like. Here, in FIG. 12, characters "incubator (1)" to "incubator (4)" are illustrated in the incubator portions 12-1 to 12-4, and numerical values (1) to (4) for identifying the incubators are indicated by using circled numbers.

In the cell culture apparatus 160 illustrated in FIG. 12, a linear motion (linear) actuator 162 is added, and four incubator portions 12-1 to 12-4 are disposed along the linear motion actuator 162 extending linearly. As the incubator portions 12-1 to 12-4, incubator portions having the same configuration can be adopted. In the example of FIG. 12, the incubator portion 12-1 is disposed at a position facing the culture medium exchange portion 22 as in the example of FIG. 1 and the like, and the incubator portions 12-2 and 12-3 are disposed to face each other with the linear motion actuator 162 interposed therebetween.

The transfer robot 40 is installed on the linear motion actuator 162 and is movable along the Y-axis direction in which the linear motion actuator 162 extends. FIG. 12 illustrates the transfer robots 40 at two positions but the number of transfer robots 40 is one, and in FIG. 12, both the transfer robots 40 before and after the movement are indicated by solid lines. Then, the transfer robot 40 takes in and out the stocker 54 with respect to each of the incubator portions 12-1 to 12-4 disposed along the linear motion actuator 162.

In such a cell culture apparatus 160, the configuration can be expanded. As a result, more cells can be cultured. Further, the cell culture apparatus 160 becomes more multi-functional. Furthermore, the position of the transfer robot 40 can be changed. The transfer robot 40 can linearly move by the linear motion actuator 162 in addition to its own six-axis operation. Therefore, this also makes the cell culture apparatus 160 more multi-functional.

Note that the configuration to be added is not limited to the incubator portion 12, and another configuration may be added. In the example of FIG. 12, the image inspection portion 14 is disposed so as to face the incubator portions 12-1 to 12-4 with the linear motion actuator 162 interposed therebetween.

### <Modification of arrangement of culture medium exchange robot 42>

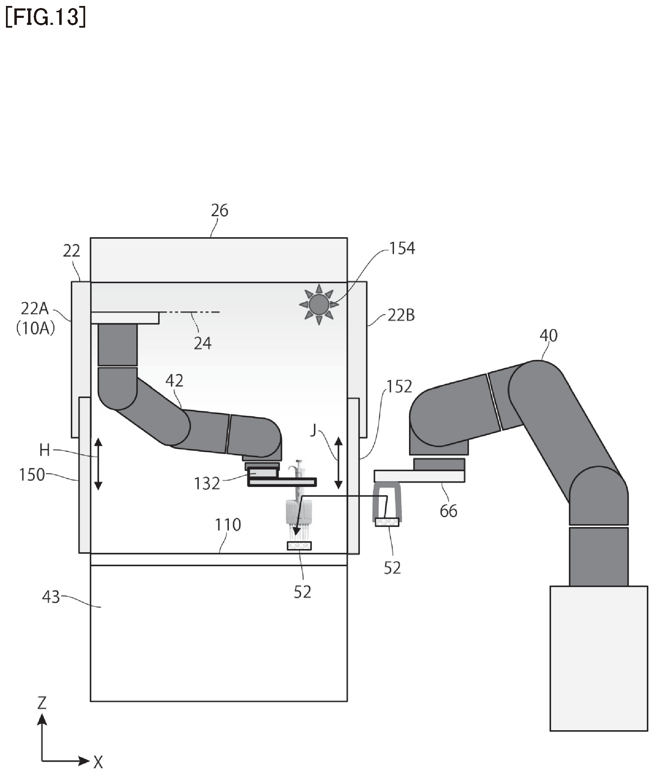

Further, in the example of FIG. 11, the culture medium exchange robot 42 is installed on the worktable 110, but the present invention is not limited thereto, and for example, as illustrated in FIG. 13, the culture medium exchange robot 42 can be a ceiling-suspended type. In the example of FIG. 13, the culture medium exchange robot 42 is installed on the inner side (internal space side) of the ceiling portion 24 of the culture medium exchange portion 22 and extends downward from the ceiling portion 24. In this way, a free space can be secured on the worktable 110, and the worktable 110 can be widely used. Further, since the space for installing the culture medium exchange robot 42 on the worktable 110 is eliminated, the worktable 110 can be formed smaller than the example of FIG. 11, and the culture medium exchange portion 22 can be downsized.

### <Supplement regarding intake of air>

In the cell culture apparatus 10 as described above, air is supplied to the internal space (first space) of the culture medium exchange portion 22 by the first space filter unit 26, and air is supplied to a space (second space) other than the internal space of the culture medium exchange portion 22 by the second space filter unit 28. Furthermore, the first space filter unit 26 takes in the air outside the cell culture apparatus 10 independently of the second space filter unit 28, and the air to the internal space (first space) of the culture medium exchange portion 22 is supplied from the first space filter unit 26 to the internal space (first space) of the culture medium exchange portion 22.

Then, the air outside the cell culture apparatus 10 can be independently taken in as the air for the internal space (first space) of the culture medium exchange portion 22 and the air for the other internal space (second space), and supplied to the internal space (first space) of the culture medium exchange portion 22 and the other internal space (second space) without being diverted at an intermediate point. Furthermore, for example, even with a simplified structure in which autoclave sterilization is not performed, air having a relatively high cleanliness can be supplied to the internal space (first space) of the culture medium exchange portion 22.

Further, since the normal environment can be maintained without depending on sterilization by steam, it is possible to maintain the inside of the cell culture apparatus at an air cleanliness suitable for the operation content without requiring measures against heat such as steam temperature management, blocking of a heat conduction (and heat transfer) path, opening and closing of a sealed door, and the like. Further, when the transfer robot 40 moves to the internal space (first space) of the culture medium exchange portion 22, it is possible to omit the step of stopping and sterilizing at an intermediate point, and this enables the transfer robot 40 to quickly move from the space (second space) outside the culture medium exchange portion 22 to the internal space (first space) of the culture medium exchange portion 22.

### <Supplement regarding function of air gripper 68>

The air gripper 68 ((a) and (b) of FIG. 7, and the like) described above can be described as follows in addition to the above description. For example, the air gripper 68 can transfer different objects by performing a partially common operation. Thus, the air gripper 68 enables the transfer robot 40 to perform various functions with a limited number of types (aspects) of operation.

Specifically, the air gripper 68 is used to transfer the stocker 54, the well plate 52, the lid 52b, and the like. The air gripper 68 performs an operation of closing the grip portion 76 as a common operation in any case of transferring an object. Furthermore, after performing such a common operation, the air gripper 68 grips a different object by a difference in the spacing between the ledge portions 78 (it is also referred to as a difference in a motion amount or a difference in a displacement amount). Thus, it is possible to transfer a large number of stockers 54 and well plates 52 using a single (one type of) air gripper 68.

More specifically, when the air gripper 68 transfers the stocker 54, as described above, the four locking pins 82 enter the respective V-shaped notches 63 provided in the ledge portions 78 and are locked to the stocker 54 in accordance with the closing operation of the grip portion 76. As a result, the stocker 54 is positioned with respect to the ledge portion 78 and gripped by the grip portion 76.

When the distal end portion of the transfer robot 40 moves upward in a state where the grip portion 76 grips the stocker 54, the air cylinder 70 is also integrally displaced. The ledge portion 78 of the grip portion 76 comes into contact with the protrusion 62 of the stocker 54 and other portions from below and ascends, and the stocker 54 is lifted. At this time, a force with which the grip portion 76 grips the stocker 54 is relatively small, and a force required for the ledge portion 78 to support the stocker 54 is larger and dominant than the force with which the grip portion 76 grips the stocker 54.

On the other hand, when the air gripper 68 grips the well plate 52 or the lid 52b, the two ledge portions 78 of the grip portion 76 gradually approach, and the four (2 × 2) resin blocks 80 come into contact with the side surfaces of the well plate body 52a or the lid 52b. Then, the well plate body 52a or the lid 52b is gripped using the frictional force accompanying the pressurization from the side surfaces without directly bringing the metal ledge portions 78 into contact with the well plate body 52a or the lid 52b.

Furthermore, when the air gripper 68 grips the well plate 52 with the lid 52b, the well plate body 52a is gripped in a state of being covered with the lid 52b, and the well plate body 52a is gripped together with the lid 52b.

When the air gripper 68 grips the well plate 52 or the lid 52b, the elevation position (position in the vertical direction (Z direction)) of the air gripper 68 and the spacing between the grip portions 76 (and the ledge portions 78) are different from those in the case of gripping the stocker 54. Further, when the well plate 52 is gripped and when the lid 52b is gripped, the elevation position of the air gripper 68 and the spacing between the grip portions 76 are different.

As described above, in the cell culture apparatus 10, it is possible to transfer a plurality of objects having different widths and heights by using the single air gripper 68 that performs the operation of closing the grip portion 76. Thus, it is possible to automate many steps required for cell culture with a simple configuration.

Furthermore, it is also possible to perform different gripping operations depending on the weight and size of the object. Then, selective use is possible such that a relatively light object (well plate 52, lid 52b, or the like) is clamped by the ledge portions 78 and a relatively heavy object (stocker 54 or the like) is supported by the ledge portions 78. The operation for such selective use is suppressed to a minimum. As a result, it is possible to grip a relatively light object and a heavy object while performing a common operation (an operation of closing or opening the grip portion 76) in many portions.

### <Utilization of commercially available laboratory instruments>

### <<Introduction>>

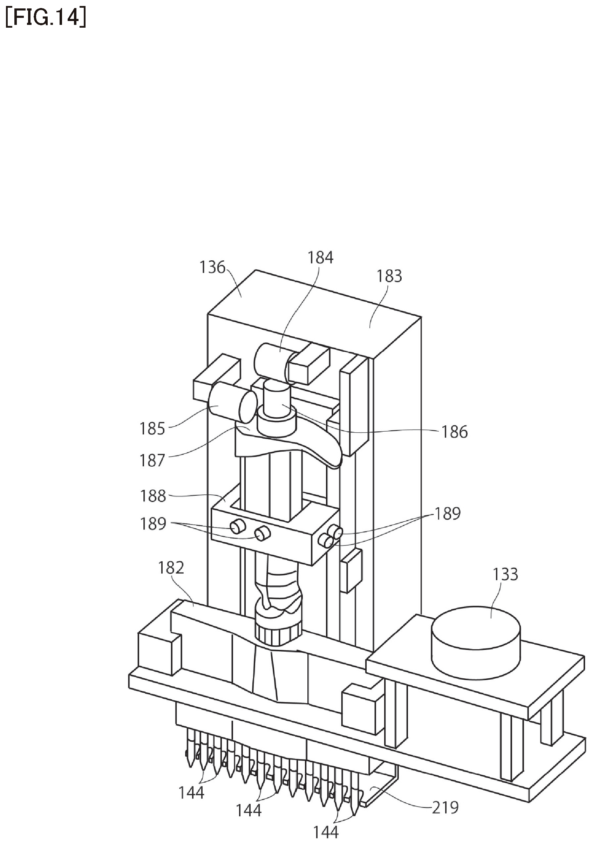

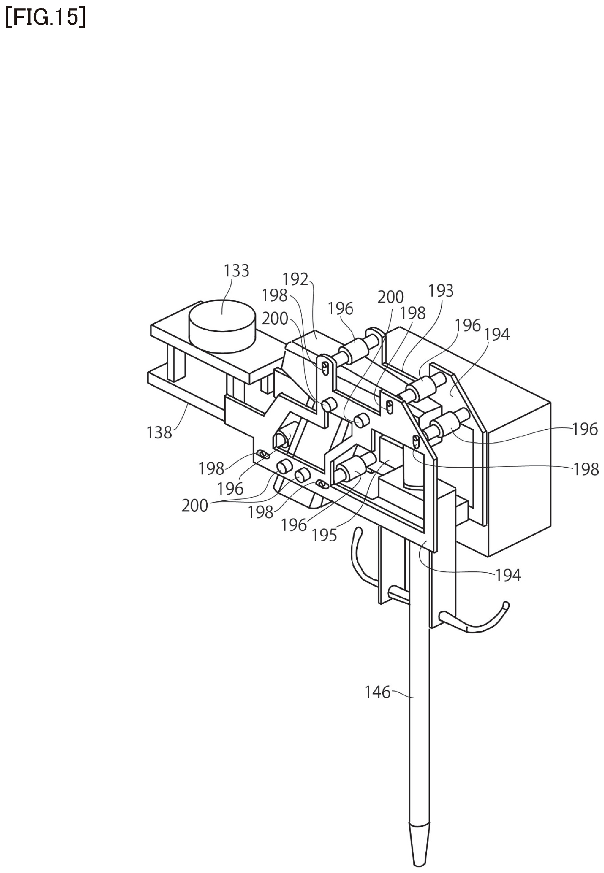

In the cell culture apparatus 10, measures are taken to enable laboratory instruments that are commercially available (commercially available laboratory instruments) to be utilized as much as possible. Examples of the commercially available laboratory instruments herein include a multi-channel micropipette 182 (FIG. 14) attached to a micropipette tool 136 (FIG. 10) and an electric pipettor 192 (FIG. 15) attached to a pipettor tool 138 (FIG. 10).

Further, examples of commercially available laboratory instruments include the distal end bit 142 of the aspirator tool 134 (FIG. 10) and a pipette tip 146 and the like attached to the electric pipettor 192 (FIG. 15). Furthermore, the reservoir 120 (FIG. 5), the incubator 32 (FIGS. 2 and 5), and the like are also included in the commercially available laboratory instruments. In addition, many commercially available laboratory instruments are used in the cell culture apparatus 10.

Commercially available laboratory instruments are supplied to the market from multiple companies and brands. However, the laboratory instruments of the same type are similar in shape, size, and the like, but are not common and are slightly different in many cases. In order to make the cell culture apparatus 10 multi-functional at low cost, it is important to be able to utilize a plurality of types of commercially available laboratory instruments regardless of differences in shape, size, and the like. In the cell culture apparatus 10, measures are taken to enable various laboratory instruments that are commercially available (commercially available laboratory instruments) to be utilized as much as possible. However, in the following, measures related to the above-described commercially available laboratory instruments will be mainly described.

### <<Measure for using multi-channel micropipette 182>>

As the multi-channel micropipette 182 (FIG. 14), a commercially available product supplied from a plurality of businesses or brands can be adopted. However, as described above, the laboratory instruments of the same type are similar in shape, size, and the like, but are not common and are slightly different in many cases.

Therefore, in order to make it possible to use commercially available laboratory instruments having different shapes, dimensions (sizes), and the like in one culture medium exchange robot 42, for example, as illustrated in FIG. 14, a driven roller 184 and an air cylinder (not illustrated) are provided in the micropipette tool 136. An air cylinder (not illustrated) is disposed inside a box 183 provided in the micropipette tool 136. A piston of an air cylinder (not illustrated) is in contact with a piston button 186 of the multi-channel micropipette 182 via a power transmission portion (not illustrated). The driven roller 184, an air cylinder (not illustrated), a power transmission portion (not illustrated), and the like constitute a push button drive mechanism portion. Then, the air cylinder (not illustrated) automatically presses the piston button 186 of the multi-channel micropipette 182. By pressing (lowering) the piston button 186, the dispensing operation is automatically performed. The piston button 186 can be pressed (lowered) by the force generated in the air cylinder (not illustrated) unless the pressing force is applied to a position separated from the piston button 186 or a position deviated to the extent of the piston button 186.

An elastic body (not illustrated) such as a coil spring is attached to the piston button 186. When released from the force generated in the air cylinder (not illustrated), the piston button 186 receives the elastic restoring force of the elastic body (not illustrated) and returns (rises) to the original position (non-pressed position). The aspiration operation is automatically performed by raising the piston button 186. The driven roller 184 follows these movements of the piston button 186.

As described above, by operating the piston button 186, the multi-channel micropipette 182, which is a commercially available laboratory instrument, can be operated even if there is a slight difference in shape, dimension (size), and the like. Then, the difference in the model of the multi-channel micropipette 182 can be absorbed. Furthermore, the multi-channel micropipettes 182 of different models can be handled by one culture medium exchange robot 42.

Note that in the example of FIG. 14, another driven roller 185 is also in contact with a shoulder portion 187 (shoulder portion of the multi-channel micropipette 182) protruding below the piston button 186. The shoulder portion 187 is also a movable component combined with an elastic body (not illustrated) such as a coil spring, and moves up and down by being pressed by a drive mechanism portion (shoulder portion drive mechanism portion) including the driven roller 185, an air cylinder (not illustrated) in the box 183, a power transmission portion (not illustrated), and the like, similarly to the piston button 186. When the shoulder portion 187 is depressed, the needle 144 attached to the multi-channel micropipette 182 automatically disengages and falls from the multi-channel micropipette 182 due to air pressure.

For example, a motor may be used instead of an air cylinder (not illustrated) that drives the piston button 186 and the shoulder portion 187. The motor may be an air motor using air as a power source or an electric motor using electricity as a power source.

The multi-channel micropipette 182 is held by a holder portion 188. In the holder portion 188, for example, different types of multi-channel micropipettes 182 having similar shapes can be held due to a difference in screwing amounts (also referred to as tightening amounts, fastening amounts, and the like) of bolts 189.

The holder portion 188 functions as a jig for holding a commercially available laboratory instrument (here, the multi-channel micropipette 182) so as to be usable. When a commercially available laboratory instrument (here, the multi-channel micropipette 182) has a curved design, it is generally difficult to incorporate the device into the micropipette tool 136. However, by adopting a jig structure such as the holder portion 188, it is possible to flexibly cope with a difference in the type of the multi-channel micropipette 182.

Note that in FIG. 14, reference numeral 133 denotes a tool-side adapter. The tool-side adapter 133 is connected to the tool adapter 132 (FIG. 10 and the like) provided at the tip of the culture medium exchange robot 42. The micropipette tool 136 (FIG. 10) attached to the culture medium exchange robot 42 is not illustrated.

### <<Measure for using electric pipettor 192>>

As the electric pipettor 192 (FIG. 15), a commercially available product supplied from a plurality of companies or brands can be adopted, but as described above, in the same type of laboratory instrument, shapes, dimensions, and the like are similar, but they are not common and are slightly different in many cases. In the pipettor tool 138 illustrated in FIG. 15, the electric pipettor 192 is held using a holder portion 193.

The holder portion 193 includes two plate-shaped frame portions 194 that are lightened, and the electric pipettor 192 is clamped between the frame portions 194. A plurality of roller portions 196 are provided between the frame portions 194, and the roller portions 196 are supported by the frame portions 194 with their end portions inserted into long holes 198. The roller portions 196 are in contact with an outer peripheral portion of the electric pipettor 192. The longitudinal direction of each of the long holes 198 faces one of two directions orthogonal to each other on the plane of the frame portions 194.

The electric pipettor 192 is surrounded by the frame portions 194 and the roller portions 196 and held by the pipettor tool 138. Since the roller portions 196 are inserted into the long holes 198, the roller portions can move within the range of the long holes 198. Thus, the positions of the roller portions 196 change depending on a difference in dimension (size) of the electric pipettor 192.

A plurality of bolts 200 is screwed into the frame portions 194. The bolts 200 are displaced forward and backward by being screwed or loosened. When the bolts 200 are screwed and advanced toward the electric pipettor 192, tips of the bolts 200 press the electric pipettor 192. When the bolts 200 are screwed, the electric pipettor 192 is firmly held as compared with that before screwing.

Note that, for example, a protective member (buffer member) made of synthetic resin (including synthetic rubber) is attached to the tip of each bolt 200, so that the bolts 200 can be prevented from damaging the electric pipettor 192. Further, as a material of the bolts 200, a synthetic resin (synthetic resin having sufficient rigidity) can also be adopted.

In this manner, the electric pipettor 192 is held by the pipettor tool 138 using the displaceable roller portions 196 and the displaceable bolts 200. The electric pipettor 192 is held at a position corresponding to a shape, a size, or the like by a displaceable member (a member capable of being displaced) such as the bolts 200 or the roller portions 196.

Therefore, the electric pipettor 192 that is a commercially available laboratory instrument can be held even if there is a slight difference in shape, dimension (size), and the like. The difference in model of the electric pipettor 192 can be absorbed. Furthermore, electric pipettors 192 of different models can be handled by one culture medium exchange robot 42.

Further, the electric pipettor 192 includes two push buttons (not illustrated), and these push buttons are pressed by two movable portions (not illustrated) provided in a button operation portion 195. In FIG. 15, the push button and the movable portion are hidden behind the frame portion 194. The functions of the two push buttons are for dispensing and for aspiration, and the two movable portions individually press the corresponding push buttons to perform dispensing operation and aspiration operation. The movable portion is driven by air pressure.

The two movable portions (not illustrated) of the button operation portion 195 can be individually driven using, for example, two air cylinders (drive sources) provided in the pipettor tool 138. In this case, the movable portion (not illustrated) can be connected to the piston of the air cylinder.

The frame portion 194 functions as a jig that holds a commercially available laboratory instrument (here, the electric pipettor 192) so as to be usable. The frame portion 194 includes the roller portions 196 and the bolts 200. When a commercially available laboratory instrument (here, the electric pipettor 192) has a curved design, it is generally difficult to incorporate the device into the pipettor tool 138. However, by adopting a jig structure such as the frame portion 194, it is possible to flexibly cope with a difference in the type of the electric pipettor 192.

Note that the electric pipettor 192 may be held while using a leaf spring force (elastic restoring force of a leaf spring) of the plate-shaped frame portion 194. Further, reference numeral 133 in FIG. 15 denotes a tool-side adapter. The tool-side adapter 133 is connected to the tool adapter 132 (FIG. 10 and the like) provided at the tip of the culture medium exchange robot 42. The pipettor tool 138 (FIG. 10) attached to the culture medium exchange robot 42 is not illustrated.

### <<Measure for using distal end bit 142 and needle 144>>

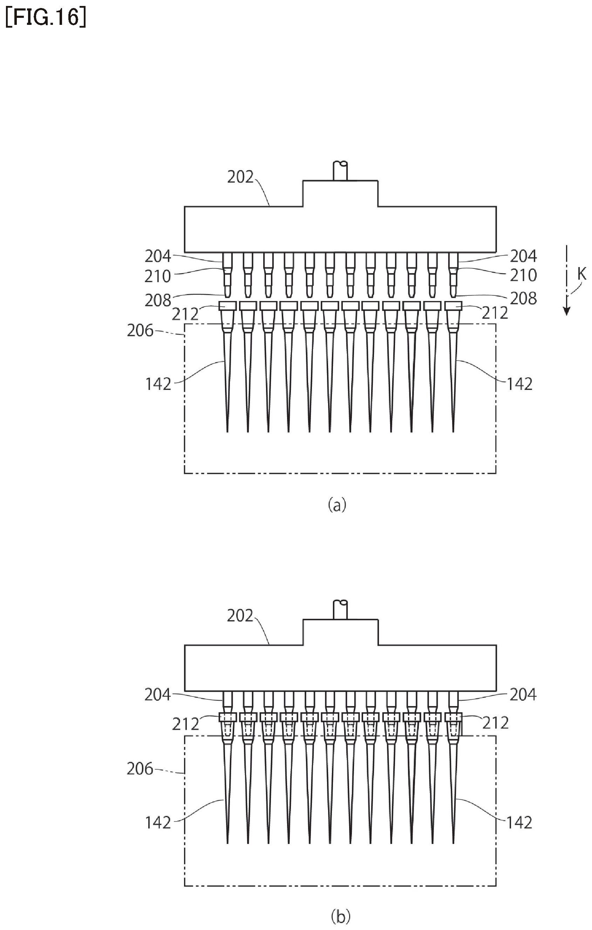

The distal end bit 142 (FIG. 10) is attached to the aspirator tool 134 (FIG. 10). A multi-aspirator 202 ((a) and (b) of FIG. 16) is attached to the aspirator tool 134 (FIG. 10), and the multi-aspirator 202 is provided with an aspirator connector 204. The distal end bit 142 is attached to the aspirator connector 204 by, for example, a procedure as illustrated in (a) and (b) of FIG. 16.

As illustrated in (a) of FIG. 16, a large number of distal end bits 142 are accommodated in a pit box 206 in a matrix arrangement. In (a) of FIG. 16, only one row of the distal end bits 142 is illustrated. One row includes 12 distal end bits 142. The aspirator tool 134 (FIG. 10) is attached to the culture medium exchange robot 42. The aspirator tool 134 (FIG. 10) attached to the culture medium exchange robot 42 is not illustrated.

The multi-aspirator 202 is attached to the aspirator tool 134. The multi-aspirator 202 is provided with a predetermined number (here, 12) of aspirator connectors 204 so as to protrude parallel to each other.

The aspirator connector 204 has tapered portions 208 and 210 at a distal end portion and an intermediate portion. The material of the aspirator connector 204 is, for example, metal such as stainless steel. Then, the culture medium exchange robot 42 lowers the multi-aspirator 202 together with the aspirator tool 134 toward the one row of distal end bits 142 as indicated by arrow K.

As illustrated in (b) of FIG. 16, the aspirator connector 204 reaches the distal end bit 142 and enters the opening at a proximal end (base portion) of the distal end bit 142. Furthermore, as the multi-aspirator 202 continues to descend, the tapered portion 210 at an intermediate portion of the aspirator connector 204 reaches the opening at the proximal end of the distal end bit 142.

The aspirator connector 204 generates a force from the inside to the outside at the proximal end portion of the distal end bit 142 by the tapered portion 210. A situation occurs in which the aspirator connector 204 is pressed against the distal end bit 142 with a predetermined force. The material of the distal end bit 142 is a synthetic resin. Then, the aspirator connector 204 is press-fitted into the distal end bit 142, and the aspirator connector 204 and the distal end bit 142 are coupled.

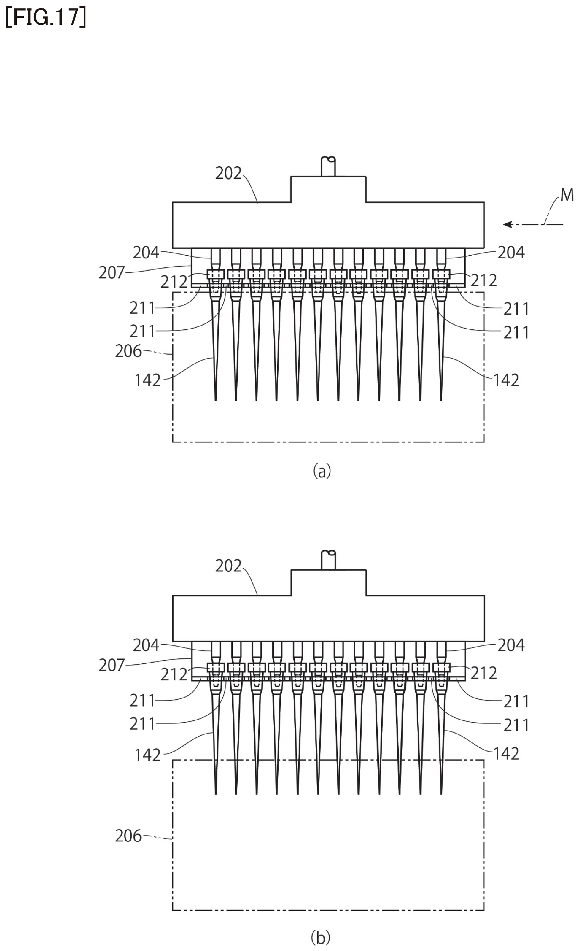

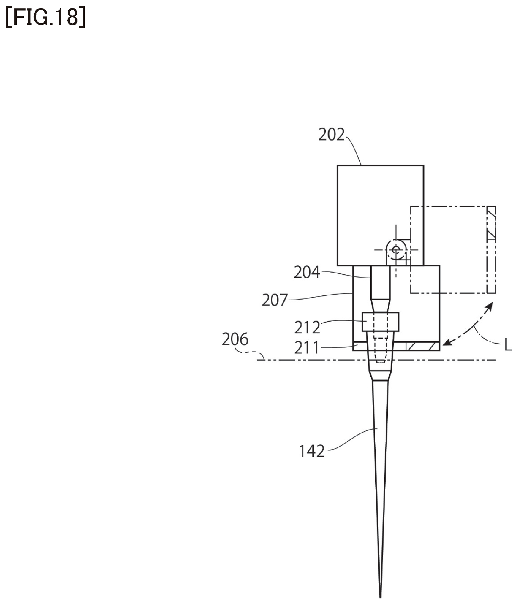

When the step of coupling the aspirator connector 204 and the distal end bit 142 is completed, the multi-aspirator 202 rises while holding the distal end bits 142 as illustrated in (b) of FIG. 17. However, prior to the rising step, a fall prevention plate 207 advances as illustrated in (a) of FIG. 17. As illustrated in FIG. 18, the term "advance" as used herein means a movement from the rear side to the front side accompanying an arc-shaped displacement as indicated by arrow L. FIG. 18 illustrates a state in which the state in (a) of FIG. 17 is viewed from the side (the direction of the arrow M in (a) of FIG. 17).

The fall prevention plate 207 constitutes a fall prevention mechanism. The fall prevention plate 207 is formed using a plate bent in an L shape. Furthermore, a plurality of claw portions 211 extending in parallel to each other is provided at an edge on a distal end side of the fall prevention plate 207, and the claw portions 211 enter between the adjacent distal end bits 142. The distal end bit 142 is provided with a flange portion 212 protruding in a tubular shape around the distal end bit. The claw portions 211 partially face, from below, the flange portion 212 protruding in a stepped shape.

Thus, for example, even if some of the aspirator connectors 204 do not sufficiently enter the corresponding distal end bits 142, the coupling is loosened, and the distal end bits 142 fall off the aspirator connectors 204, the distal end bits 142 can be hooked on the claw portions 211. Thus, it is possible to prevent the distal end bits 142 from falling onto the worktable 110 (FIG. 5).

In a case where a commercially available laboratory instrument is used as the distal end bit 142, depending on the shape, size, and the like of the distal end bit 142 used, or an angle (posture) in a situation of being accommodated in the pit box 206, it is conceivable that the multi-aspirator 202 rises while being insufficiently coupled to the aspirator connector 204. In a case where the distal end bit 142 is dropped, depending on the situation, it is necessary to stop the automatic drive of the cell culture apparatus 10, and the operator A (FIG. 1 and the like) enters the cell culture apparatus 10 (or inserts an arm) to remove the distal end bit 142 from the cell culture apparatus 10.

Further, in the cell culture apparatus 10, once one driving (operation) is started, a process necessary for cell culture may be executed continuously for 10 days or more, for example. For this reason, the dropping of the distal end bit 142 may adversely affect cell culture over the past several days. Further, the degree of cleanliness of the air may decrease due to opening of doors and shutters.

However, as illustrated in (a) and (b) of FIG. 17, by preventing the distal end bit 142 from falling, the cell culture apparatus 10 can be operated while maintaining the optimized environment without stopping the process necessary for cell culture.

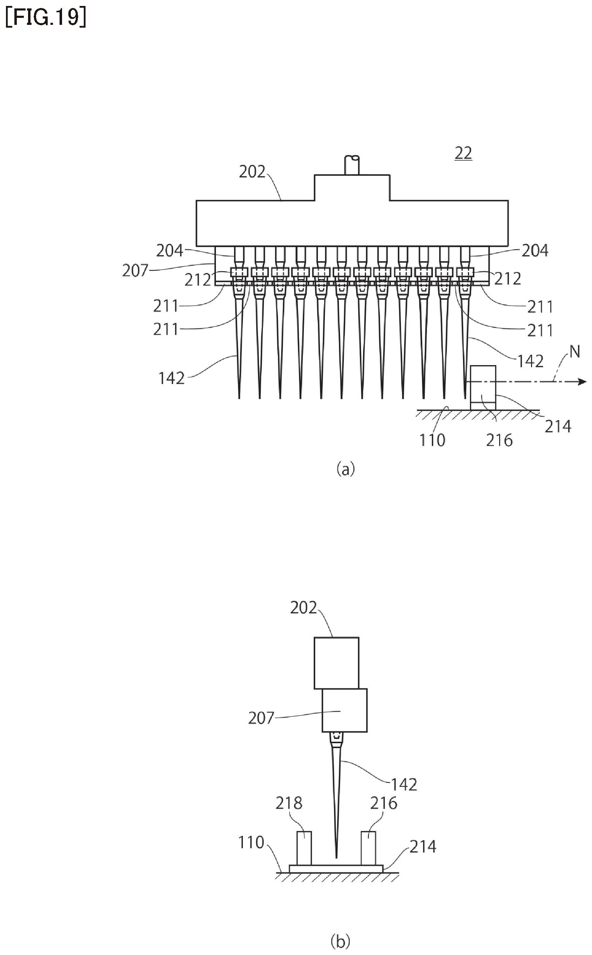

Further, as illustrated in FIG. 19, a distal end bit detection sensor 214 capable of detecting the presence or absence of falling is provided without stopping preventing the falling of the distal end bit 142 by the fall prevention mechanism including the fall prevention plate 207. As the distal end bit detection sensor 214, for example, an optical sensor including a light projecting portion 216 and a light receiving portion 218 can be adopted.

In order to detect whether the distal end bits 142 have dropped, for example, the multi-aspirator 202 holding one row of the distal end bits 142 is moved toward the distal end bit detection sensor 214 as indicated by arrow N by the culture medium exchange robot 42. The distal end bit detection sensor 214 is disposed in the culture medium exchange portion 22 similarly to the culture medium exchange robot 42.

The culture medium exchange robot 42 linearly moves the multi-aspirator 202 at a constant speed so that the distal end bits 142 sequentially pass between the light projecting portion 216 and the light receiving portion 218. The distal end bit detection sensor 214 can be installed, for example, on the worktable 110. However, the present invention is not limited thereto, and for example, it is also possible to provide a shelf (not illustrated) or the like at a position higher than the worktable 110 and install the distal end bit detection sensor 214 on the shelf.

When there is no drop, a predetermined number (12 in this embodiment) of the distal end bits 142 intermittently and regularly block light between the light projecting portion 216 and the light receiving portion 218. However, when there is a drop, the period during which the distal end bits 142 are not detected becomes longer than when there is no drop. Thus, by monitoring a waveform related to an output signal of the light receiving portion 218 by, for example, the control portion 46 (FIG. 5) or the like, it is possible to determine whether or not the distal end bits 142 have fallen and the position where the distal end bit has fallen. Here, the distal end bit detection sensor 214 may be of a type that detects reflected light from the distal end bits 142.

Note that, here, the distal end bit 142 attached to the aspirator tool 134 (FIG. 10) have been described, but similar attachment mechanisms, attachment methods, fall prevention structures, and fall prevention methods can also be adopted for the needle 144 of the micropipette tool 136. The micropipette tool 136 in the example of FIG. 14 is provided with a fall prevention plate 219 similarly to the aspirator tool 134 (FIG. 10, FIG. 17, and FIG. 18). Further, a needle detection sensor (not illustrated) can be provided. Furthermore, the distal end bit detection sensor 214 can be an optical sensor also used as a needle detection sensor.

### <<Measure for using pipette tip 146>>

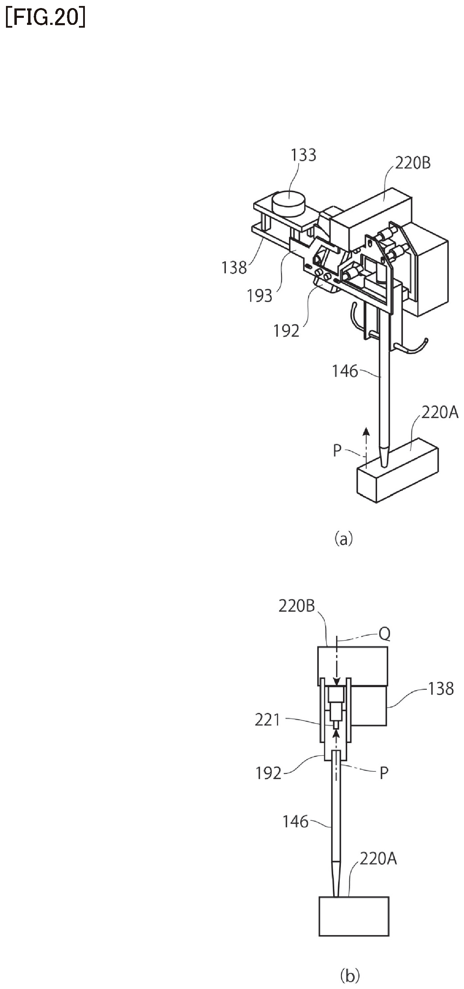

The pipette tip 146 (FIG. 10) is a large and long commercially available laboratory instrument as compared with the distal end bit 142 and the needle 144. The pipette tip 146 is attached to the electric pipettor 192 (FIG. 15). The electric pipettor 192 (FIG. 15) is held by the pipettor tool 138. Attachment of the pipette tip 146 to the electric pipettor 192 is performed by, for example, a procedure illustrated in (a) and (b) of FIG. 20.

In (a) of FIG. 20, one pipette tip 146 is held by a movable mechanism portion 220A. The movable mechanism portion 220A is connected to a drive source such as an air cylinder, and can raise the pipette tip 146 at a predetermined speed (for example, about several cm to 10 cm/sec) as indicated by arrow P. As the pipette tip 146 rises, it approaches the electric pipettor 192.

The pipette tip 146 comes into contact with a tip connector 221 of the electric pipettor 192 and continues to rise. Then, relatively the tip connector 221 enters the pipette tip 146. Furthermore, the tip connector 221 is press-fitted into the pipette tip 146, and the pipette tip 146 is connected to the electric pipettor 192.

Here, in the examples of (a) and (b) of FIG. 20, the movable mechanism portion 220A is illustrated as being in contact with the pipette tip 146 simply from below and pushing up the pipette tip 146. However, the present invention is not limited thereto, and the movable mechanism portion 220A can grip and lift the pipette tip 146 from the horizontal direction.

At this time, a pressurizing mechanism portion 220B is in contact with the electric pipettor 192 from above. The pressurizing mechanism portion 220B is formed of, for example, a member such as an aluminum profile, and moves so as to approach or move away from the electric pipettor 192. In the examples of (a) and (b) of FIG. 20, the pressurizing mechanism portion 220B is moved so as to be in contact with the electric pipettor 192 from above.

The pressurizing mechanism portion 220B receives force of the electric pipettor 192 pressed upward by the pipette tip 146 and generates downward force on the electric pipettor 192 as indicated by arrow Q in (b) of FIG. 20. The pressurizing mechanism portion 220B may apply a force from above to the electric pipettor 192 or may only contact the electric pipettor 192. Even if the pressurizing mechanism portion 220B is merely in contact with the electric pipettor 192, a force from below the movable mechanism portion 220A is transmitted to the electric pipettor 192 via the pipette tip 146 to generate a reaction force.

As a result, it is possible to generate a force for causing the tip connector 221 to enter the upper opening of the pipette tip 146. Then, the pipette tip 146 can be attached to the electric pipettor 192 while sufficiently securing a rising force of the pipette tip 146 (a pushing up force of the movable mechanism portion 220A).

In other words, by supporting the electric pipettor 192 from above with the pressurizing mechanism portion 220B, it is possible to supplement (augment or increase) the force of attaching the pipette tip 146 to the electric pipettor 192. Furthermore, since the pressurizing mechanism portion 220B receives the force by which the movable mechanism portion 220A rises, the load applied to the culture medium exchange robot 42 can be minimized.

There is no need to increase the size or load of the movable mechanism portion 220A or the drive system of the movable mechanism portion 220A. That is, the drive system of the movable mechanism portion 220A can be reduced in size with a low load. Furthermore, it is not necessary to introduce a dedicated large device in order to attach the pipette tip 146 to the electric pipettor 192. Thus, the cell culture apparatus 10 can be downsized.

### <<Measure for using incubator 32>>

As for the incubator 32, opening and closing of the door in a commercially available incubator is automated. Furthermore, in the automation, it is not necessary to modify the incubator, and an inexpensive and compact configuration is achieved.

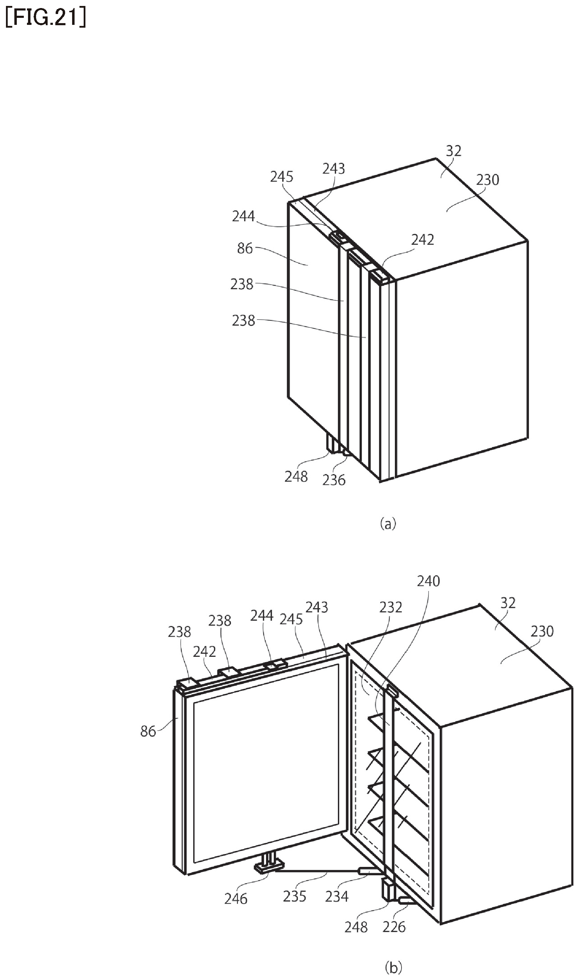

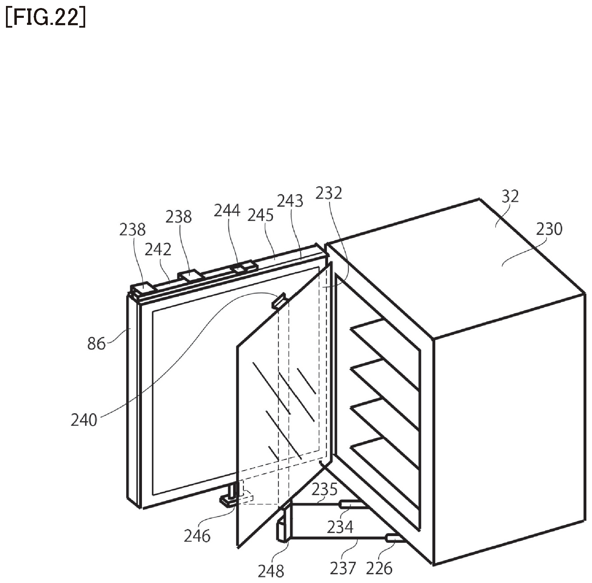

As described above, the incubator 32 (FIGS. 2 and 5) is provided with the automatic door 86. The automatic door 86 is a one-side hinge-type door and horizontally turns (rotationally displaces) while being supported by an incubator body 230. FIG. 21 illustrates, in (a), a state in which the automatic door 86 is closed, and illustrates, in (b), a state in which the automatic door 86 is opened. The automatic door 86 functions as an outer door of the incubator 32. Furthermore, as illustrated in (b) of FIG. 21 and FIG. 22, an inner door 232 is provided inside the automatic door 86. The inner door 232 is also a one-side hinge type door and is turned (rotated and displaced) in the horizontal direction in a state of being supported by the incubator body 230.

The automatic door 86 is formed by bending a sheet metal into a rectangular box shape, and coating or the like is applied to the entire automatic door 86. Thus, the inside of the incubator 32 cannot be visually recognized through the automatic door 86. On the other hand, a transparent resin, glass, or the like is used for the inner door 232. When only the inner door 232 is closed, the inside of the incubator 32 can be visually recognized through the inner door 232. As described above, the incubator 32 has a double door structure.

For example, the automatic door 86 and the inner door 232 are individually opened and closed under the control of the control portion 46 (FIG. 5). As illustrated in (b) of FIG. 21 and FIG. 22, piston rods 235 and 237 of air cylinders 234 and 236 are connected to the automatic door 86 and the inner door 232. The automatic door 86 and the inner door 232 are connected to the piston rods 235 and 237 using band plate-shaped holding members 238 and 240. The number of holding members 238 of the automatic door 86 is two, and the number of holding members 238 of the inner door 232 is one. The portions where the automatic door 86 and the inner door 232 are connected to the piston rods 235 and 237 are fixing portions 246 and 248, respectively.

The holding members 238 and 240 are disposed outside the automatic door 86 and the inner door 232 with the longitudinal direction thereof oriented in the vertical direction. The end portions of the holding members 238 and 240 in the longitudinal direction (vertical direction) have a bent shape. Then, the holding members 238 and 240 are attached to the automatic door 86 and the inner door 232 via a clamping structure (clamp structure) that clamps the upper and lower sides of the automatic door 86 and the inner door 232. Note that, specifically, the holding members 238 and 240 are formed by combining an L-shaped sheet metal member or a resin plate member with a plate-shaped aluminum frame.

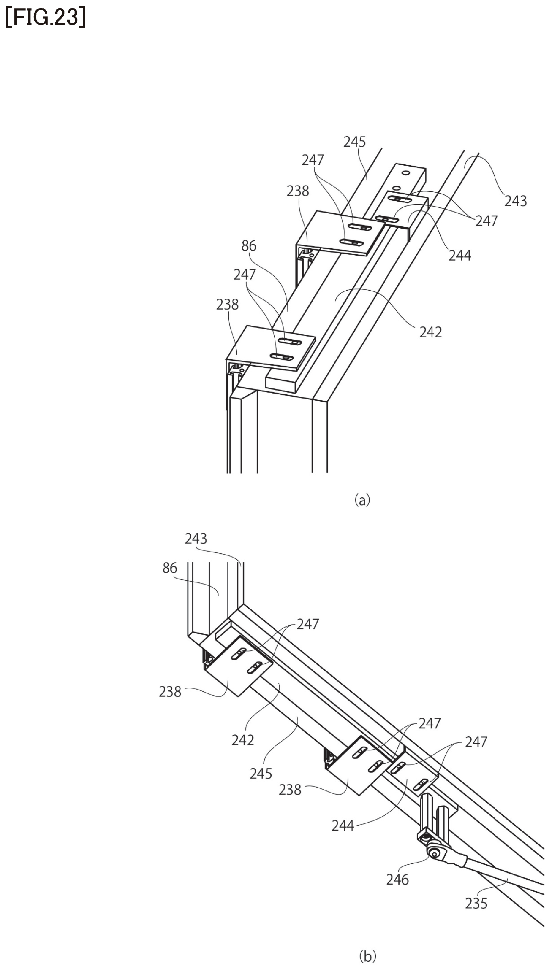

The two holding members 238 of the automatic door 86 are coupled to each other by a coupling plate (bracket) 242. As illustrated in (a) and (b) of FIG. 23, the holding member 238 is screwed to the automatic door 86 in a state of being overlapped with the coupling plate 242 from the outside. The coupling plate 242 is screwed to the automatic door 86 in a state of being overlapped with the automatic door 86.

The holding member 238 and the coupling plate 242 are fixed to the automatic door 86 by screwing screws into existing holes in the automatic door 86. Furthermore, as illustrated in (a) and (b) of FIG. 23, an L-shaped bracket 244 is inserted and fixed in a gap between an existing door seal 243 of the automatic door 86 and a frame 245. A long hole 247 is formed at the end portion of the holding member 238, and the fixing position can be adjusted within the range of the long hole 247. By using the holding member 238, the coupling plate 242, and the L-shaped bracket 244, a force for clamping the automatic door 86 from the vertical direction by the holding member 238 and a force for clamping the automatic door 86 from the thickness direction (clamping force) by the holding member 238 and the L-shaped bracket 244 can be generated. With such a structure, even when the number of existing holes in the automatic door 86 is small (for example, one or the like) and a fixing force by screwing is small, it is possible to sufficiently secure the fixing force for transmitting the force of the air cylinder 234 to the automatic door 86.

The piston rod 235 of the air cylinder 234 is coupled to a coupling plate 242 provided along the lower side of the automatic door 86 ((b) of FIG. 23). The piston rod 235 is coupled to the coupling plate 242 in a state where relative displacement in the horizontal direction is possible about the pivot of the fixing portion 246. Although not illustrated, the proximal end side of the air cylinder 234 connected to the incubator body 230 can also be relatively displaced in the horizontal direction about the pivot.

As illustrated in FIG. 22, an end portion of the holding member 238 of the inner door 232 in the longitudinal direction protrudes upward and downward of the inner door 232. The piston rod 237 of the air cylinder 236 corresponding to the inner door 232 is connected to a fixing portion 248 formed at a lower end portion of the holding member 238. Although not illustrated in detail, the piston rod 237 is connected to the holding member 238 in a state where relative displacement in the horizontal direction is possible about the pivot of the fixing portion 248. Although not illustrated, the proximal end side of the air cylinder 236 connected to the incubator body 230 can also be relatively displaced in the horizontal direction about the pivot.

The automatic door 86 and the inner door 232 are opened by being pushed by the air cylinders 234 and 236 and are closed by being pulled by the air cylinders 234 and 236. In FIG. 22, both the automatic door 86 and the inner door 232 are opened at different opening degrees.

As the air cylinders 234 and 236, closed-center air cylinders are used. In the closed-center air cylinders 234 and 236, the positions of the piston rods 235 and 237 are maintained in an intermediate position state. In the example of (a) in FIG. 21, the piston rods 235 and 237 are in the intermediate position state. In the example of FIG. 22, the piston rod 235 connected to the automatic door 86 is in a state of protruding more than the intermediate position state, and the piston rod 237 connected to the inner door 232 is in the intermediate position state.

The displacement speed of the piston rods 235 and 237 is adjusted using a speed controller. The thrust of the piston rods 235 and 237 is adjusted using a regulator (pneumatic regulator).

The incubator 32 is one of commercially available laboratory instruments, and when the commercially available incubator 32 is used, the automatic door 86 and the inner door 232 are conventionally opened and closed manually. However, in the cell culture apparatus 10, the opening and closing of the automatic door 86 and the inner door 232 are automated, and the opening and closing of the door of the incubator 32, which is a commercially available laboratory instrument, are automated.

The air cylinders 234 and 236 are controlled so as to reproduce how the automatic door 86 and the inner door 232 are opened and closed by a human in a process necessary for cell culture. Further, air for driving the air cylinders 234 and 236 is not air used in the culture medium exchange robot 42, for example, but is introduced by an independent air system.

Note that in the incubator 32 illustrated in the examples of FIGS. 21 to 23, the inner door 232 is provided with a manual lock mechanism. However, since it is necessary to provide more devices in order to automate the operation of the lock mechanism, the lock mechanism is removed in the examples of FIGS. 21 to 23. Note that it is also possible to leave the lock mechanism without removing it and keep it in an unused state. Furthermore, the lock mechanism may be automated as long as the operation of the lock mechanism can be performed by a simple device.

The items related to the incubator 32 as described above can be organized as follows.
(1) In the incubator 32, the air cylinders 234 and 236 are used as drive sources for door opening and closing (opening and closing of the automatic door 86 and/or the inner door 232).
(2) The air cylinders 234 and 236 are compactly disposed outside (here, below) the incubator 32.
(3) No separate link mechanism is combined with the air cylinders 234 and 236, and the doors (automatic door 86 and inner door 232) and the air cylinders 234 and 236 are directly connected at the fixing portions 246 and 248. The doors (automatic door 86 and inner door 232) and the air cylinders 234 and 236 are directly connected by the fixing portions 246 and 248, and the air cylinders 234 and 236 themselves serve as links. Thus, it is possible to dispose a mechanical configuration for automatically opening and closing the doors (automatic door 86 and inner door 232) in a space-saving manner.
(4) The air cylinders 234 and 236 are closed-center air cylinders, and the closed state of the automatic door 86 (and/or the inner door 232) is maintained when the piston rod 235 (and/or the piston rod 237) is in the intermediate position state. Thus, it is not necessary to separately provide a lock mechanism.
(5) Opening and closing speeds of the doors (automatic door 86 and inner door 232) can be individually adjusted using the speed controller, and a thrust for opening and closing the door can be adjusted using a regulator (air pressure regulator). Since an air device (pneumatic device) is used, the opening and closing of the door can be easily adjusted and controlled by the speed controller and the regulator. Thus, the configuration for opening and closing the doors (automatic door 86 and inner door 232) can be achieved at low cost.
(6) The holding members 238 and 240 are attached to the doors (automatic door 86 and inner door 232) by a clamping structure, and the holding members 238 and 240 are fixed to the air cylinders 234 and 236. The reason why the doors (automatic door 86 and inner door 232) and the drive portion (here, air cylinders 234 and 236) are fixed in this manner is as follows. That is, when a commercially available incubator is used, a large modification such as changing (exchanging) a rotation shaft of the incubator or making a hole in a door is not preferable because performance guarantee by a manufacturer of the incubator or the like cannot be received or cost increases.
(7) The automatic door 86 and the inner door 232 are driven by two independent air cylinders 234 and 236, respectively. In many commercially available incubators, the door has a two-door configuration including an outer door and an inner door. The outer door is opaque and has high heat insulation performance. Further, the inner door is made of glass or a transparent resin. As described above, by adopting the two-door configuration, there are an advantage that a heat loss at the time of opening and closing the door is small, an advantage that the inside can be checked without opening the inner door, and the like. In the cell culture apparatus 10, the door is opened in the order of the outer door (automatic door 86) and the inner door 232, and the door is closed in the order of the inner door 232 and the outer door (automatic door 86).

### <<Summary of use of commercially available laboratory instrument>>

As described above, various commercially available laboratory instruments are utilized. In the cell culture apparatus 10, a commercially available laboratory instrument manually operated by a human is incorporated in an automated system and used.

Note that it is possible to hold a commercially available laboratory instrument not only in the culture medium exchange robot 42 but also in the transfer robot 40, for example. Regarding the transfer robot 40, for example, commercially available products can be used as the well plate 52 and the lid 52b. In this case, it can be said that the air gripper 68 is provided with a measure that allows a plurality of types of commercially available laboratory instruments to be utilized by a partially common operation.

### <Drive mode of shutter>

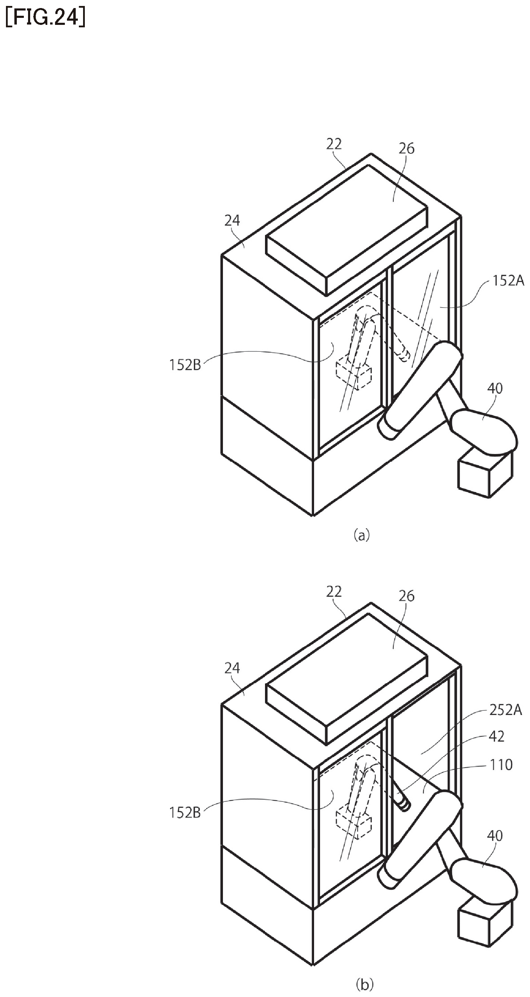

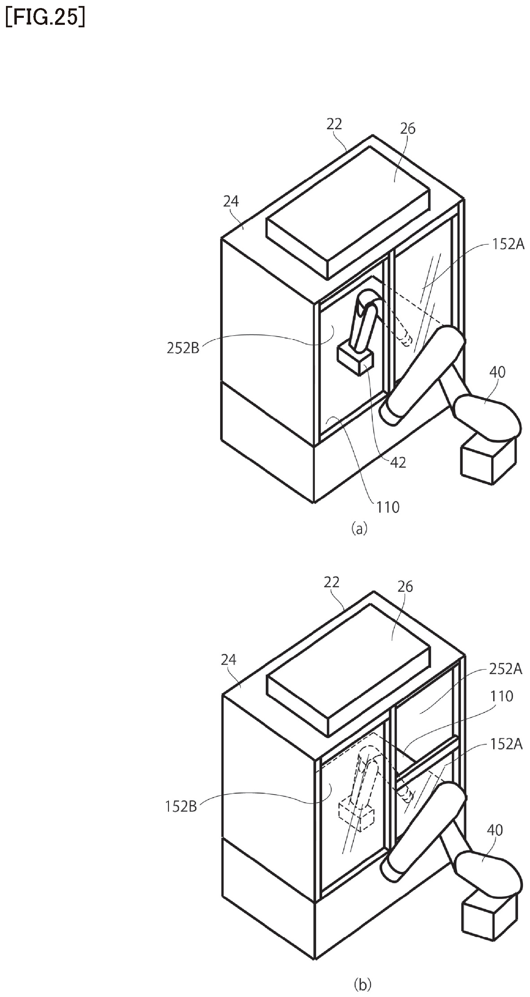

With respect to the shutter mechanism such as the automatic opening and closing shutter 152 (FIG. 11) described above, it is possible to enhance a sealing effect as much as possible by making an opening area adjustable. Specifically, for example, as illustrated in (a) of FIG. 24, two automatic opening and closing shutters 152A and 152B are disposed side by side in the lateral direction (lateral direction, horizontal direction). As illustrated in (b) of FIG. 24 and (a) of FIG. 25, the automatic opening and closing shutters 152A and 152B are individually installed in openings 252A and 252B of the culture medium exchange portion 22. The automatic opening and closing shutters 152A and 152B constitute an inner wall portion 22B of the culture medium exchange portion 22 and face the transfer robot 40.

The automatic opening and closing shutters 152A and 152B are individually controlled for opening and closing under the control of the control portion 46 (FIG. 5), for example. As an example, as illustrated in (b) of FIG. 24, one automatic opening and closing shutter 152A is opened. Although not illustrated, in this state, the transfer robot 40 causes the arm plate 66 (FIG. 10) to enter the culture medium exchange device 43 through the opening 252A. After finishing the operation using the air gripper 68 in the culture medium exchange region 157 (FIG. 5), the transfer robot 40 moves the arm plate 66 (FIG. 10) to the outside of the culture medium exchange device 43.

One of the opened automatic opening and closing shutters 152A is lifted, the opening 252A is closed, and the other closed automatic opening and closing shutter 152B is opened as illustrated in (a) of FIG. 25. Although not illustrated, the transfer robot 40 causes the fork portion 84 (two long claw portions) of the arm plate 66 (FIG. 10) to face the culture medium exchange device 43 and enter the culture medium exchange device 43 through the opening 252B.

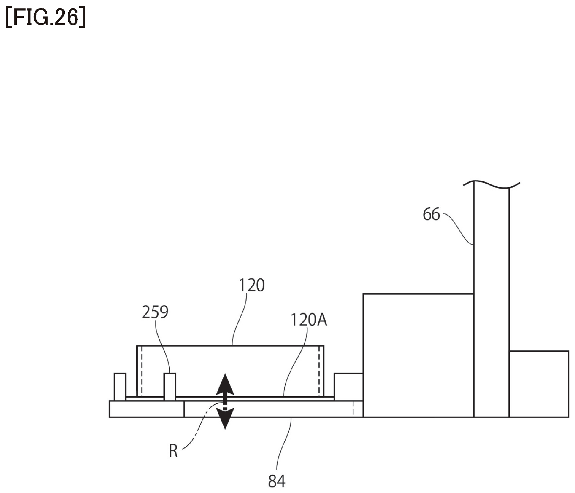

Thereafter, the transfer robot 40 supports one reservoir 120 located at the uppermost stage among the plurality of reservoirs 120 stacked in the reservoir preparation region 158 and places the reservoir 120 in the reservoir placement region 128. Prior to the placement of the reservoir 120 in the reservoir placement region 128, the transfer robot 40 performs an operation (swinging operation) of moving the arm plate 66 up and down in a short cycle as illustrated in FIG. 26, which will be described later.

The transfer robot 40 moves the arm plate 66 (FIG. 10) to the outside of the culture medium exchange device 43. Then, the opened automatic opening and closing shutter 152B is lifted and closed, and both the automatic opening and closing shutters 152A and 152B are closed.

The amount (degree of opening, opening degree) by which the automatic opening and closing shutters 152A and 152B are opened can be individually and arbitrarily changed between 0 (fully closed) and 100% (fully opened). Thus, the automatic opening and closing shutters 152A and 152B can be opened with the minimum necessary opening degree. Then, the opening areas of the openings 252A and 252B can be finely adjusted to be minimized.

FIG. 25 illustrates, in (b), a state in which the opening degree of one automatic opening and closing shutter 152A (and the opening 252A) is set to about 50% and the opening degree of the other automatic opening and closing shutter 152B (and the opening 252B) is set to 0%.

For example, as illustrated in (a) of FIG. 25, with the opening degree of the automatic opening and closing shutter 152B set to 100% and the longitudinal direction of the arm plate 66 (FIG. 10) directed vertically, the arm plate 66 (FIG. 10) can be made to enter the opening 252B. Furthermore, as illustrated in (b) of FIG. 25, with the opening degree of the automatic opening and closing shutter 152A set to 50% and the longitudinal direction of the arm plate 66 (FIG. 10) directed forward and backward (front and back directions in the horizontal direction), the arm plate 66 (FIG. 10) can be made to enter the opening 252A.

Then, it is possible to finely adjust and minimize the area of the opening in the same plane by a combination of opening and closing and opening degrees of the two automatic opening and closing shutters 152A and 152B arranged in the lateral direction. Furthermore, it is possible to minimize the area of the opening through which the transfer robot 40 to which the arm plate 66 is attached enters and exits. Here, the "area of the opening" can be adjusted for both the area of each of the openings 252A and 252B and the total area.

Note that the internal space (first space) of the culture medium exchange device 43 is kept at a positive pressure by the air from the first space filter unit 26. Thus, the air in the second space does not immediately enter by the opening of the automatic opening and closing shutters 152A and 152B. However, since the transfer robot 40 and the arm plate 66 enter and exit, it is effective to minimize the opening areas of the openings 252A and 252B.

Further, since the automatic opening and closing shutters 152A and 152B are displaced up and down, it is possible to prevent the automatic opening and closing shutters 152A and 152B from interfering with devices, instruments, and the like in the culture medium exchange device 43. Further, the space in the vertical direction of the cell culture apparatus 10 can be effectively used, and the culture medium exchange device 43 and the cell culture apparatus 10 can be downsized with respect to the left, right, front, and rear (Y direction and X direction in FIGS. 1 and 2). Note that the number of the automatic opening and closing shutters 152A and 152B may be one or three or more. Further, fixed window portions (fixed window), columns, and the like may be provided between the automatic opening and closing shutters 152A and 152B, and it can be said that the automatic opening and closing shutters 152A and 152B are arranged side by side in the lateral direction including such cases.

### <Gripping of one reservoir 120>

As described above, the transfer robot 40 performs an operation (swinging operation) of moving the arm plate 66 up and down in a short period. With this operation, the transfer robot 40 reliably supports one reservoir 120 located at the uppermost stage among the plurality of reservoirs 120 stacked in the reservoir preparation region 158.

The reservoir 120 is molded with a certain degree of flexibility using a synthetic resin material. The degree of flexibility is so that elastic deformation in appearance can be performed manually. Such flexibility is obtained not only by the properties of the synthetic resin material but also by the wall thickness of the reservoir 120. Further, each of the reservoirs 120 is molded, for example, in a container shape opened in a rectangular shape. The reservoirs 120 have the same shape and are stacked in a state in which the unevenness is fitted to each other to some extent.

From these factors, there is a case where the reservoirs 120 stacked in the reservoir preparation region 158 are in close contact with the next (lower) reservoir, and the upper and lower reservoirs 120 are stuck to each other. Furthermore, three or more reservoirs 120 may be stuck together. However, the arm plate 66 ascends while gripping one reservoir 120 at the uppermost stage and moves up and down in a short period (for example, 1 to 2 times/second) as indicated by arrow R in FIG. 26.

The gripping by the arm plate 66 (also referred to as gripping by the transfer robot 40) is performed by sandwiching a gripping target object (here, the reservoir 120) between some of the gripping pins 259 (only one side is illustrated in FIG. 26) provided in the fork portion 84. The gripping pin 259 is formed in a columnar shape and vertically protrudes from the fork portion 84. When the reservoir 120 is gripped, the flange portion 120A formed on the peripheral edge of the reservoir 120 is supported by the fork portion 84 from below.

By performing such a swinging operation, the reservoirs 120 in the second and subsequent stages from the top that are not directly gripped are shaken off. As a result, only the uppermost reservoir 120 can be gripped, and only one reservoir 120 can be reliably placed in the reservoir placement region 128.

Note that, for example, it is also possible to detect whether or not there is only one reservoir 120 in the reservoir placement region 128. More specifically, although not illustrated, an optical sensor including a light projector and a light receiver is placed in the reservoir placement region 128. The optical sensor is disposed so that the reservoir 120 is located therebetween. If there is a foreign object (here, the reservoir 120) at a position exceeding the height of one reservoir 120, detection light of the optical sensor is blocked. Then, the control portion 46 determines that the plurality of reservoirs 120 remain overlapped on the basis of the interruption of light detection. Note that the optical sensor may be of a type that detects reflected light from the reservoir 120.

### <Structure for maintaining air cleanliness>

### <<Structure for disposal to disposal box 114>>

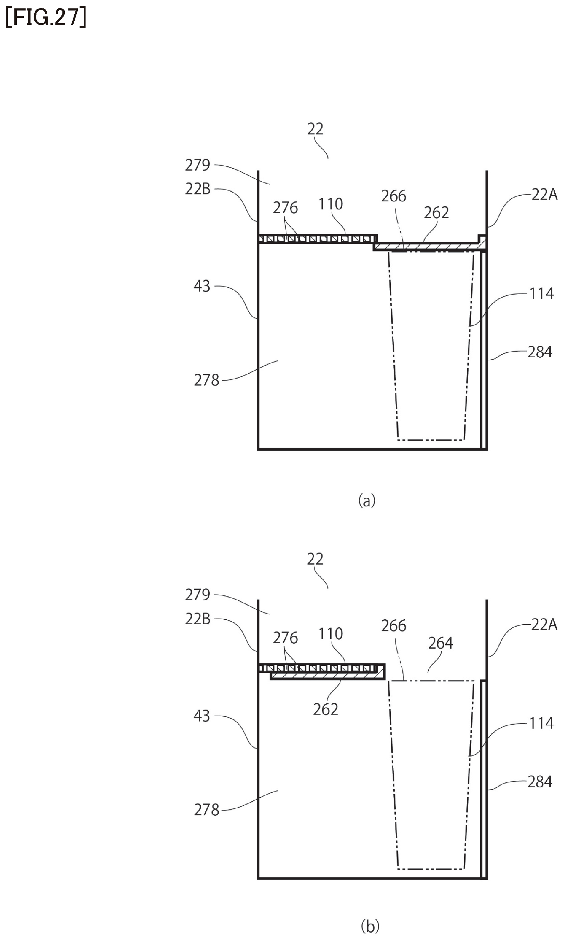

Inside the culture medium exchange device 43, upper and lower spaces are partitioned by the worktable 110. As illustrated in (a) and (b) of FIG. 27, the worktable 110 is provided with a disposal shutter 262. FIG. 27 illustrates, in (a) and (b), the lower part of the culture medium exchange portion 22 partially longitudinally.

The disposal shutter 262 is normally closed as illustrated in (a) of FIG. 27. However, when the disposal box 114 is used, as illustrated in (b) of FIG. 27, the disposal shutter 262 slides in the horizontal direction (Y direction in FIG. 1) to expose an internal disposal port 264. Sliding of the disposal shutter 262 can be automatically performed under the control of the control portion 46 (FIG. 5).

The size of the internal disposal port 264 is substantially the same as an opening 266 of one disposal box 114. For example, the distal end bits 142 of the aspirator tool 134 (FIG. 10) and the like are discarded in the disposal box 114. The removal of the distal end bits 142 can be performed using air pressure. Further, in the micropipette tool 136 (FIG. 10, FIG. 14), the needle 144 removed by pressing the shoulder portion 187 of the multi-channel micropipette 182 can be dropped into the disposal box 114 and disposed of.

Further, normally, the disposal shutter 262 is closed, and the liquid such as the culture solution is prevented from falling into the disposal box 114. For example, the disposal shutter 262 is automatically controlled for opening and closing by the control portion 46 (FIG. 5).

In this manner, it is possible to automate the operation of disposal to the disposal box 114 by providing the disposal shutter 262. Then, for example, the air cleanliness of the surrounding environment of the culture medium exchange robot 42 can be maintained as compared with a case where the operator opens the culture medium exchange portion 22 and manually discards the culture medium.

### <<Structure partitioning inside of culture medium exchange device 43>>

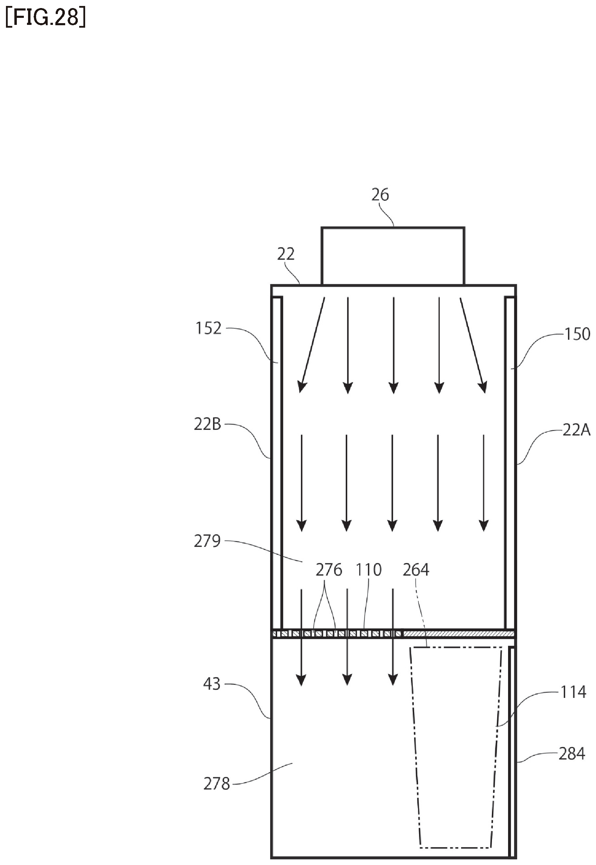

A metal plate member made of a stainless material or the like is used for the worktable 110. As illustrated in FIG. 28, a punching panel having a large number of through holes 276 is employed as at least a part of the plate material of the worktable 110. Then, the air from the first space filter unit 26 flows (downflows) toward the worktable 110, passes through the through holes 276 of the worktable 110, and flows into a lower space 278.

In an upper space 279 of the worktable 110, an operation by the culture medium exchange robot 42 is performed, and in the lower space 278, the disposal box 114 and the like are installed. The air in the environment around the culture medium exchange robot 42 is discharged from the upper space 279 to the lower space 278. Furthermore, the air in the lower space 278 is discharged from the culture medium exchange device 43 to the outside of the cell culture apparatus 10.

As described above, a space having a relatively high cleanliness (upper space 279) and a space having a relatively low cleanliness (lower space 278) are partitioned inside the culture medium exchange device 43. Then, the operation by the culture medium exchange robot 42 is performed in a space having a relatively high cleanliness (upper space 279). Further, since the air from the first space filter unit 26 flows toward the worktable 110, this also prevents the air in the lower space 278 from returning to the upper space 279. From these facts, it is possible to maintain the air cleanliness of the surrounding environment of the culture medium exchange robot 42.

### <<Structure for opening and closing lower space 278>>

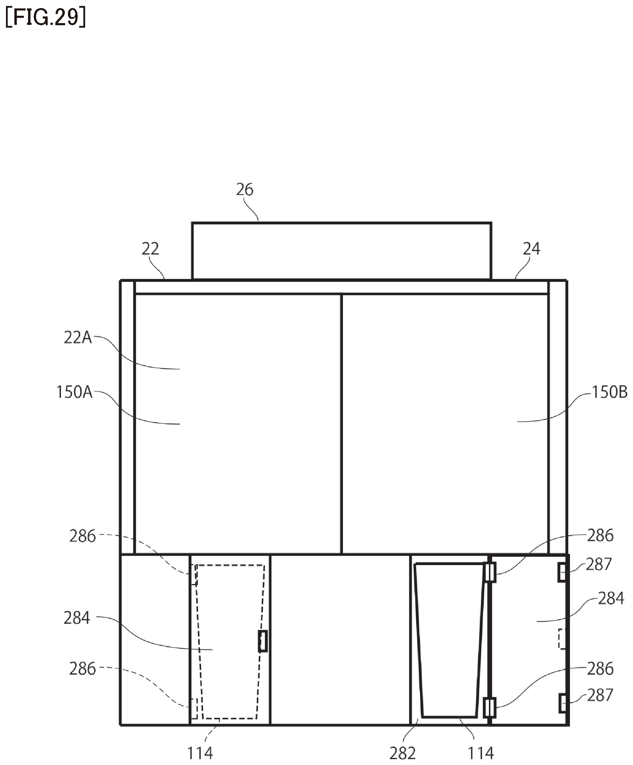

As illustrated in FIG. 29, two external disposal ports 282 are provided in an openable and closable manner in a lower portion of the outer wall portion 22A of the culture medium exchange portion 22. The external disposal port 282 is interposed between the lower space 278 in the culture medium exchange portion 22 and the external space of the culture medium exchange portion 22 (here, the external space of the cell culture apparatus 10).

Normally, as illustrated on the left side in the figure, the external disposal port 282 is closed by an external disposal port door 284. However, when the disposal box 114 is exchanged, the external disposal port door 284 is opened as illustrated on the right side in the figure. Then, the disposal box 114 is taken in and out through the external disposal port 282. At least a part of the air in the lower space 278 is ventilated when the external disposal port door 284 is opened. The external disposal port door 284 illustrated on the left side in the figure can be similarly opened and closed.

The external disposal port door 284 is supported by the culture medium exchange portion 22 so as to be openable and closable by a hinge 286. Furthermore, the closed state of the external disposal port door 284 is maintained by a latch mechanism 287. The external disposal port door 284 is manually opened and closed.

### <Discharge of contaminated fluid>

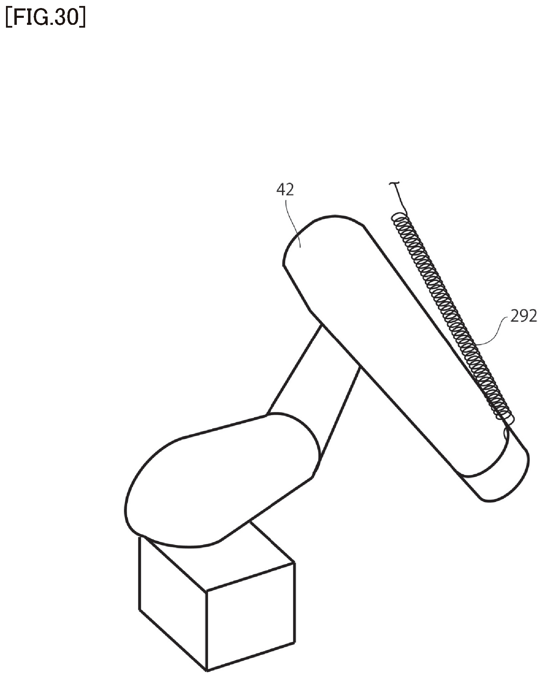

As described above, the aspirator tool 134 (FIG. 10) attached to the culture medium exchange robot 42 aspirates the old culture medium from the well of the well plate body 52a (FIG. 6 and the like). As illustrated in FIG. 30, the old culture medium aspirated from the distal end bits 142 of the aspirator tool 134 is collected in a waste liquid tube 292 provided in the culture medium exchange robot 42, passes through the waste liquid tube 292, and is collected from the distal end side to the proximal end side of the culture medium exchange robot 42. Further, the aspirator tool 134 may also aspirate cleaning alcohol from the distal end bits 142 for cleaning.

The waste liquid tube 292 is a flexible spiral tube (spiral pipe) and is made of synthetic resin. Most of the waste liquid tube 292 is disposed not inside the culture medium exchange robot 42 but outside the culture medium exchange robot 42. By using the spiral tube as the waste liquid tube 292, the waste liquid tube 292 is located outside the culture medium exchange robot 42, and the culture medium exchange robot 42 can operate in a large range. That is, the waste liquid tube 292 does not inhibit the operation of the culture medium exchange robot 42. Further, the waste liquid tube 292 is not pulled by excessive tension due to the operation of the culture medium exchange robot 42. An upper end side of the waste liquid tube 292 (the proximal end side of the culture medium exchange robot 42) can be supported by the ceiling portion 24 of the culture medium exchange portion 22. Furthermore, the waste liquid tube 292 is exchanged after the end portion is removed from the culture medium exchange robot 42 by operation (manual operation) from outside the culture medium exchange robot 42. Thus, at the time of exchange of the waste liquid tube 292, it is not necessary to perform an operation of opening a maintenance opening (not illustrated) of the culture medium exchange robot 42 and taking out most of the waste liquid tube 292 from the inside to the outside of the culture medium exchange robot 42. Then, the number of man-hours required for the exchange operation can be suppressed.

Liquid piping, air piping, and the like are provided inside the culture medium exchange robot 42. The waste liquid tube 292 is more likely to be contaminated than other pipes and is exchanged more frequently. Then, if the exchange operation is performed by opening the maintenance opening (not illustrated) of the culture medium exchange robot 42 and taking it out, many man-hours are required. Therefore, by disposing the waste liquid tube 292 outside the culture medium exchange robot 42, the waste liquid tube 292 can be easily exchanged.

### <Invention derivable from embodiments>

The following are derivable from the embodiments described so far.
(1) A cell culture apparatus including:
   a first space (such as an internal space of the culture medium exchange portion 22) having air cleanliness that satisfies a first criterion (such as Class 5 according to ISO) as a standard criterion thereof;
   a second space (such as an internal space of a portion other than the culture medium exchange portion 22) having air cleanliness that satisfies a second criterion (such as Classes 6 to 7 according to ISO) as a standard criterion thereof, the second criterion being lower than the first criterion;
   a first operation portion (such as a culture medium exchange robot 42) that is provided in the first space, the first operation portion being configured to execute an operation (such as aspiration or discharge of a culture solution) related to culture medium exchange;
   a second operation portion (such as the transfer robot 40) that is provided in the second space, the second operation portion being configured to execute a moving operation of a cell culture container (such as the well plate 52) that stores a culture medium; and
   an operation portion controller (such as the control portion 46) that is configured to set, as the first space, a movable range in a case where the first operation portion executes the operation related to the culture medium exchange, and set, as the second space and the first space, a movable range in a case where the second operation portion moves the cell culture container.
   This allows the movable ranges of the first operation portion and the second operation portion to overlap, enabling downsizing of the cell culture apparatus. Furthermore, the operation portions having different functions can be operated in a more suitable environment.
(2) The cell culture apparatus according to (1), wherein the operation portion controller is configured to control the second operation portion to move the cell culture container from the second space to the first space and control the first operation portion to execute the operation related to the culture medium exchange for the cell culture container. This enables downsizing of the cell culture apparatus.
(3) The cell culture apparatus according to (2), wherein the cell culture container comprises a dustproof portion (lid 52b or the like) attached and detached by the second operation portion. This enables the cell culture container to be kept clean.
(4) The cell culture apparatus according to any one of (1) to (3), wherein the second operation portion includes a gripping mechanism (such as air gripper 68) that is configured to grip the cell culture container, and the first operation portion includes an attaching mechanism (such as tool adapter 132) that is common among a plurality of types of operation devices (an aspirator tool 134, micropipette tool 136, pipettor tool 138, and the like) corresponding to content of the operation related to the culture medium exchange. This enables easy automation of gripping of the cell culture container and attachment of the operation device.
(5) The cell culture apparatus according to any one of (1) to (4), including, in the second space, a supply and discharge portion (stocker supply portion 16 and product removal portion 18, or stocker supply and removal portion in which both are integrated, and the like) in which the cell culture container is loaded and unloaded; a cell culture portion (such as incubator portion 12) in which cells in the cell culture container are cultured; and an inspection portion (such as image inspection portion 14) in which an inspection of the cell culture container is performed. The operation portion controller is configured to control the second operation portion to perform a moving operation of the cell culture container with respect to the supply and discharge portion, a moving operation of the cell culture container with respect to the cell culture portion, and a moving operation of the cell culture container with respect to the inspection portion. This enables downsizing of the cell culture apparatus.
(6) The cell culture apparatus according to any one of (1) to (5), wherein central portions (central portions (16+18)C, 12C, 14C, and the like) of the first space, the supply and discharge portion, the cell culture portion, and the inspection portion are separately disposed in at least three quadrants (at least three quadrants among first quadrant Q1 to fourth quadrant Q4 or the like) in an orthogonal coordinate system (XYZ coordinates or the like) centered on the second operation portion in plan view. This enables downsizing of the cell culture apparatus.
(7) The cell culture apparatus according to any one of (1) to (6), further including a germicidal light source portion (sterilization lamp (or germicidal lamp) 154 or the like) that is configured to sterilize the first space. This enables the internal environment of the first space to be kept clean.
(8) The cell culture apparatus according to any one of (1) to (7), further including a moving portion (linear motion actuator 162 or the like) that is configured to move the second operation portion. This enables the cell culture apparatus to be more multi-functional.
(9) The cell culture apparatus according to (4) to (8), further including: a container placement portion (such as a stocker 54) on which the cell culture container is placed, wherein the gripping mechanism includes a ledge portion (such as ledge portion 78) whose spacing is changeable, and the gripping mechanism is configured to grip the cell culture container and the container placement portion by changing the spacing of the ledge portion. This enables easy automation of gripping and transfer of the cell culture container and the container placement portion.
(10) The cell culture apparatus according to (9), wherein the cell culture containers are stacked on the container placement portion to form a stack (a set of up to five well plates 52), and the container placement portion is gripped by the gripping mechanism in a state where the plurality of (three or the like) stacks are arranged on the container placement portion.
   This enables simultaneous transfer of a large number of cell culture containers.
(11) The cell culture apparatus according to any one of (1) to (10), further including a stop condition detection portion (human detection sensor 159 or the like) that is configured to detect a stop condition (entry of a person or the like) that is a condition for stopping the second operation portion. The operation portion controller is configured to operate the first operation portion and stop the second operation portion when the stop condition is detected. This enables safer operation of a small cell culture apparatus.
(12) The cell culture apparatus according to any one of (1) to (11), wherein air is supplied to the first space by a first space filter unit (first space filter unit 26 or the like); and air is supplied to the second space by a second space filter unit (second space filter unit 28 or the like). The first space filter unit is configured to independently take in air outside the cell culture apparatus with respect to the second space filter unit, and air to the first space is supplied from the first space filter unit to the first space. This enables air outside the cell culture apparatus to be independently taken in as air for the first space and air for the second space and supplied to the first space and the second space without being divided at an intermediate point.
(13) The cell culture apparatus according to any one of (1) to (12), further including a shutter member (automatic opening and closing shutters 152A, 152B, and the like) that is configured to partition the first space and the second space; and a shutter member control portion (such as controller 46) that is configured to control opening and closing of the shutter member, and an opening degree of the shutter member. An area of an opening (each of openings 252A and 252B, the sum of openings 252A and 252B, or the like) between the first space and the second space is adjustable by a combination of the opening and closing of the shutter member, and the opening degree of the shutter member. This enables the shutter member to be opened with a necessary minimum opening degree.
(14) The cell culture apparatus according to (13), wherein a plurality of the shutter members are provided, and the plurality of shutter members are arranged side by side in a lateral direction. This enables minimization, as much as necessary, of the area of the opening in the same plane.
(15) The cell culture apparatus according to (13) or (14), wherein the second operation portion is configured to move the cell culture container between the second space and the first space via the opening. This enables minimization, as much as necessary, of the area of the opening through which the second operation portion enters and exits.
(16) The cell culture apparatus according to any one of (1) to (15), further including, in the first space, a worktable portion (such as worktable 110) on which a cell culture container is placeable. The worktable portion includes a plurality of air passage holes (such as through holes 276) through which air supplied from the first space filter unit to the first space passes. This enables the first space to be partitioned into a space having a relatively high cleanliness (upper space 279) and a space having a relatively low cleanliness (lower space 278).
(17) The cell culture apparatus according to (4) to (16), wherein the operation device (micropipette tool 136, pipettor tool 138, and the like) includes a holder portion (holder portion 188 for multi-channel micropipette 182, holder portion 193 for electric pipettor 192, and the like) to which different laboratory instruments (multi-channel micropipette 182, electric pipettor 192, and the like) are attachable. This enables utilization of different types of commercially available laboratory instruments.
(18) The cell culture apparatus according to (4) to (17), wherein the operation device (such as micropipette tool 136) includes a push button drive mechanism portion (driven rollers 184 and 185, air cylinder, power transmission portion, and the like) that is configured to operate a push button (piston button 186, shoulder portion 187, and the like) of a different laboratory instrument. This enables utilization of different types of commercially available laboratory instruments including the push button.
(19) The cell culture apparatus according to (4) to (18), wherein a laboratory instrument (multi-aspirator 202, multi-channel micropipette 182, and the like) is attachable to the operation device, the laboratory instrument is configured to automatically attach a plurality of tubular devices (distal end bit 142, needle 144, and the like), and the operation device includes a fall prevention mechanism (such as a fall prevention mechanism including a fall prevention plate 207) that is configured to prevent the tubular devices from falling. This enables prevention of the tubular device from falling.
(20) The cell culture apparatus according to (4) to (19), wherein a laboratory instrument (such as electric pipettor 192) is attachable to the operation device (such as pipettor tool 138). The laboratory instrument is configured to automatically attach a tubular device (such as a pipette tip 146), and the cell culture apparatus further includes an attaching force reinforcing portion (such as pressurizing mechanism portion 220B). The attaching force reinforcing portion is configured to support the laboratory instrument from a direction opposite to a direction in which the tubular device is pressed against the laboratory instrument when the tubular device is attached to the laboratory instrument (such as when the movable mechanism portion 220A attaches the pipette tip 146 to the electric pipettor 192), and compensate for a force by which the tubular device is pressed against the laboratory instrument. This enables the tubular device to be attached to the laboratory instrument with a small force.
(21) The cell culture apparatus according to (1) to (20), further including an incubator (such as an incubator 32) in the second space, the incubator including a door (automatic door 86, inner door 232, and the like) that is configured to open and close in a horizontal direction, and an air cylinder (air cylinders 234, 236, and the like) that is configured to generate a force for opening and closing the door. This enables a commercially available incubator to be used.
(22) The cell culture apparatus according to (1) to (21), wherein at least one of the first operation portion and the second operation portion is configured to perform a shaking operation in a vertical direction while gripping a reservoir (such as the reservoir 120) in the first space. This enables one reservoir to be accurately gripped.
(23) The cell culture apparatus according to (1) to (22), further including, in the first operation portion, a waste liquid tube (waste liquid tube 292 or the like) through which a waste liquid (old culture medium or the like) passes. The waste liquid tube is provided outside the first operation portion. This enables the waste liquid tube to be removed without an operation of taking out the waste liquid tube from the first operation portion.

### <Others>

Note that the present invention is not limited to the above-described various embodiments, and various modifications and combinations of various embodiments may be made without departing from the gist of the invention.

For example, in organoid culture, the culture medium exchange may be performed by a mechanism or method similar to that of the culture medium exchange portion 22 of the above-described embodiment.

### Industrial Applicability

A cell culture apparatus according to the present invention can be applied to various cell cultures in which a culture medium is exchanged.

### Cross-Reference to Related Applications

The present application claims priority to Japanese Patent Application No. 2023-214803 filed with the Japan Patent Office on December 20, 2023, the entire disclosure of which is incorporated herein by reference in its entirety.

### Reference Signs List

- 10: Cell culture apparatus
- 10A: Wall portion
- 10B: Window portion
- 12: Incubator portion
- 14: Image inspection portion
- 16: Stocker supply portion
- 18: Product removal portion
- 20: Transfer robot portion
- 22: Culture medium exchange portion
- 26: First space filter unit
- 28: Second space filter unit
- 32: Incubator
- 34: Image inspection apparatus
- 36: Opening and closing shutter portion
- 40: Transfer robot
- 42: Culture medium exchange robot
- 46: Control portion
- 52: Well plate
- 52a: Well plate body
- 52b: Lid
- 54: Stocker
- 56: Well
- 68: Air gripper
- 70: Air cylinder
- 72: Movable body
- 76: Grip portion
- 78: Ledge portion
- 80: Resin block
- 82: Locking pin
- 90: Camera
- 120: Reservoir
- 130: Tool changer
- 132: Tool adapter
- 134: Aspirator tool
- 136: Micropipette tool
- 138: Pipettor tool
- 142: Distal end bit
- 144: Needle
- 146: Pipette tip
- 148: Reception plate
- 152A, 152B: Automatic opening and closing shutter
- 154: Sterilization lamp
- 159: Human detection sensor
- 182: Micropipette
- 184, 185: Driven roller
- 186: Piston button
- 187: Shoulder portion
- 188, 193: Holder portion
- 189: Bolt
- 192: Electric pipettor
- 194: Frame portion
- 195: Button operation portion
- 196: Roller portion
- 198: Long hole
- 200: Bolt
- 202: Multi-aspirator
- 204: Aspirator connector
- 206: Pit box
- 207: Fall prevention plate
- 208, 210: Tapered portion
- 211: Claw portion
- 212: Flange portion
- 214: Distal end bit detection sensor
- 216: Light projecting portion
- 218: Light receiving portion
- 219: Fall prevention plate
- 220A: Movable mechanism portion
- 220B: Pressurizing mechanism portion
- 221: Tip connector
- 232: Inner door
- 234, 236: Air cylinder
- 235, 237: Piston rod
- 238, 240: Holding member
- 242: Coupling plate
- 243: Door seal
- 244: L-shaped bracket
- 245: Frame
- 246, 248: Fixing portion
- 247: Long hole
- 252A, 252B: Opening
- 259: Gripping pin
- 262: Disposal shutter
- 264: Internal disposal port
- 266: Opening
- 276: Through hole
- 278: Lower space
- 279: Upper space
- 282, 284: External disposal port
- 286: Hinge
- 287: Latch mechanism
- 292: Waste liquid tube

## Claims

1. A cell culture apparatus comprising:
a first space having air cleanliness that satisfies a first criterion as a standard criterion thereof;
a second space having air cleanliness that satisfies a second criterion as a standard criterion thereof, the second criterion being lower than the first criterion;
a first operation portion that is provided in the first space, the first operation portion being configured to execute an operation related to culture medium exchange;
a second operation portion that is provided in the second space, the second operation portion being configured to execute a moving operation of a cell culture container that stores a culture medium; and
an operation portion controller that is configured to
set, as the first space, a movable range in a case where the first operation portion executes the operation related to the culture medium exchange, and
set, as the second space and the first space, a movable range in a case where the second operation portion moves the cell culture container.

2. The cell culture apparatus according to claim 1, wherein the operation portion controller is configured to
control the second operation portion to move the cell culture container from the second space to the first space, and
control the first operation portion to execute the operation related to the culture medium exchange for the cell culture container.

3. The cell culture apparatus according to claim 2, wherein the cell culture container comprises a dustproof portion attached and detached by the second operation portion.

4. The cell culture apparatus according to any one of claims 1 to 3, wherein
the second operation portion comprises a gripping mechanism that is configured to grip the cell culture container, and
the first operation portion comprises an attaching mechanism that is common among a plurality of types of operation devices corresponding to content of the operation related to the culture medium exchange.

5. The cell culture apparatus according to claim 1, comprising, in the second space,
a supply and discharge portion in which the cell culture container is loaded and unloaded;
a cell culture portion in which cells in the cell culture container are cultured; and
an inspection portion in which an inspection of the cell culture container is performed;
wherein the operation portion controller is configured to control the second operation portion to perform a moving operation of the cell culture container with respect to the supply and discharge portion, a moving operation of the cell culture container with respect to the cell culture portion, and a moving operation of the cell culture container with respect to the inspection portion.

6. The cell culture apparatus according to claim 5, wherein central portions of the first space, the supply and discharge portion, the cell culture portion, and the inspection portion are separately disposed in at least three quadrants in an orthogonal coordinate system centered on the second operation portion in plan view.

7. The cell culture apparatus according to any one of claims 1 to 3, further comprising a germicidal light source portion that is configured to sterilize the first space.

8. The cell culture apparatus according to any one of claims 1 to 3, further comprising a moving portion that is configured to move the second operation portion.

9. The cell culture apparatus according to claim 4, further comprising:
a container placement portion on which the cell culture container is placed,
wherein the gripping mechanism comprises a ledge portion whose spacing is changeable, and
the gripping mechanism is configured to grip the cell culture container and the container placement portion by changing the spacing of the ledge portion.

10. The cell culture apparatus according to claim 9, wherein the cell culture containers are stacked on the container placement portion to form a stack, and the container placement portion is gripped by the gripping mechanism in a state where the plurality of stacks are arranged on the container placement portion.

11. The cell culture apparatus according to any one of claims 1 to 3, further comprising:
a stop condition detection portion that is configured to detect a stop condition that is a condition for stopping the second operation portion,
wherein the operation portion controller is configured to operate the first operation portion and stop the second operation portion when the stop condition is detected.

12. The cell culture apparatus according to any one of claims 1 to 3, wherein
air is supplied to the first space by a first space filter unit;
air is supplied to the second space by a second space filter unit;
the first space filter unit is configured to independently take in air outside the cell culture apparatus with respect to the second space filter unit, and
air to the first space is supplied from the first space filter unit to the first space.

13. The cell culture apparatus according to any one of claims 1 to 3, further comprising:
a shutter member that is configured to partition the first space and the second space; and
a shutter member control portion that is configured to control opening and closing of the shutter member, and an opening degree of the shutter member,
wherein an area of an opening between the first space and the second space is adjustable by a combination of the opening and closing of the shutter member, and the opening degree of the shutter member.

14. The cell culture apparatus according to claim 13, wherein a plurality of the shutter members are provided, and the plurality of shutter members are arranged side by side in a lateral direction.

15. The cell culture apparatus according to claim 13, wherein the second operation portion is configured to move the cell culture container between the second space and the first space via the opening.

16. The cell culture apparatus according to any one of claims 1 to 3, further comprising, in the first space, a worktable portion on which a cell culture container is placeable,
wherein the worktable portion comprises a plurality of air passage holes through which air supplied from the first space filter unit to the first space passes.

17. The cell culture apparatus according to claim 4, wherein the operation device comprises a holder portion to which different laboratory instruments are attachable.

18. The cell culture apparatus according to claim 4, wherein the operation device comprises a push button drive mechanism portion that is configured to operate a push button of a different laboratory instrument.

19. The cell culture apparatus according to claim 4, wherein
a laboratory instrument is attachable to the operation device,
the laboratory instrument is configured to automatically attach a plurality of tubular devices, and
the operation device comprises a fall prevention mechanism that is configured to prevent the tubular devices from falling.

20. The cell culture apparatus according to claim 4, wherein
a laboratory instrument is attachable to the operation device,
the laboratory instrument is configured to automatically attach a tubular device, and
the cell culture apparatus further comprises an attaching force reinforcing portion, and
the attaching force reinforcing portion is configured to
support the laboratory instrument from a direction opposite to a direction in which the tubular device is pressed against the laboratory instrument when the tubular device is attached to the laboratory instrument, and
compensate for a force by which the tubular device is pressed against the laboratory instrument.

21. The cell culture apparatus according to any one of claims 1 to 3, further comprising:
an incubator in the second space, the incubator comprising a door that is configured to open and close in a horizontal direction, and
an air cylinder that is configured to generate a force for opening and closing the door.

22. The cell culture apparatus according to any one of claims 1 to 3, wherein at least one of the first operation portion and the second operation portion is configured to perform a shaking operation in a vertical direction while gripping a reservoir in the first space.

23. The cell culture apparatus according to any one of claims 1 to 3, further comprising, in the first operation portion, a waste liquid tube through which a waste liquid passes,
wherein the waste liquid tube is provided outside the first operation portion.
